# NEW EUROPEAN PATENT SPECIFICATION

(11) **EP 2 522 744 B2**
(45) Date of publication and mention of the opposition decision: **23.01.2019**
(45) Mention of the grant of the patent: 31.12.2014
(21) Application number: 12167334.7
(22) Date of filing: 09.05.2012
(51) Int. Cl.: C12Q 1/68

(54) **A canine BCAN microdeletion associated with Episodic Falling Syndrome**
Mit dem EFS (Episodic Falling Syndrome) assoziierte BCAN-Mikrodeletion beim Hund
Microdéletion du gène canin BCAN associée au syndrome de chute épisodique

(30) Priority: 11.05.2011 EP 11165620
(43) Date of publication of application: 14.11.2012
(73) Proprietor: Laboklin GmbH & Co. KG, 97688 Bad Kissingen (DE); UCL Business PLC, London Greater London W1T 4TP (GB)
(72) Inventor: Harvey, Robert Justin, Letchworth Garden City Hertfordshire SG6 2BT (GB); Gill, Jennifer Louise, London SW11 1PG (GB); Clark, Leigh Anne, Clemson, SC 29631 (US)
(74) Representative: advotec.

(56) References cited:
- WO-A2-03/007880
- WO-A2-2005/069852
- BROWNE D L ET AL: "EPISODIC ATAXIA/MYOKYMIA SYNDROME IS ASSOCIATED WITH POINT MUTATIONS IN THE HUMAN POTASSIUM CHANNEL GENE, KCNA1", NATURE GENETICS, NATURE PUBLISHING GROUP, NEW YORK, US, vol. 8, 1 January 1994 (1994-01-01), pages 136-140, XP000647694, ISSN: 1061-4036, DOI: 10.1038/NG1094-136
- JEFF S DAVIES ET AL: "The glycinergic system in human startle disease: a genetic screening approach", FRONTIERS IN MOLECULAR NEUROSCIENCE, vol. 3, 1 January 2010 (2010-01-01), pages 1-10, XP55032259, DOI: 10.3389/fnmol.2010.00008
- C. BRAKEBUSCH ET AL: "Brevican-Deficient Mice Display Impaired Hippocampal CA1 Long-Term Potentiation but Show No Obvious Deficits in Learning and Memory", MOLECULAR AND CELLULAR BIOLOGY, vol. 22, no. 21, 1 November 2002 (2002-11-01), pages 7417-7427, XP55032286, ISSN: 0270-7306, DOI: 10.1128/MCB.22.21.7417-7427.2002
- JENNIFER L. GILL ET AL: "A canine BCAN microdeletion associated with episodic falling syndrome", NEUROBIOLOGY OF DISEASE, vol. 45, no. 1, 28 July 2011 (2011-07-28), pages 130-136, XP55032256, ISSN: 0969-9961, DOI: 10.1016/j.nbd.2011.07.014
- <<<N O N - C I T E D D O C U M E N T>>> Galtrey: "The role of chondroitin sulfate proteoglycans in regeneration and plasticity in the central nervous system", BRAIN RESEARCH REVIEWS, vol. 54, 31 March 2007 (2007-03-31), pages 1-18, XP022011311,
- <<<N O N - C I T E D D O C U M E N T>>> KARLSSON, E.: "Efficient mapping of mendelian traits in dogs through genome-wide association", Nature Genetics, vol. 39, no. 11, 1 November 2007 (2007-11-01), pages 1321-1328, XP055032283,
- <<<N O N - C I T E D D O C U M E N T>>> ARCHIVED WEBSITE CAPTURE OF 'HTTP://CAVALIEREPISODICFALLING.COM/RESEAR CH' ON MARCH 27,2009 (OBTAINED VIA WAYBACKMACHINE).
- <<<N O N - C I T E D D O C U M E N T>>> MEURS, K.: "Genome-wide association identifies a deletion in the 3' untranslated region of Striatin in a canine model of arrhythmogenic right ventricular cardiomyopathy", Human Genetics, vol. 128, no. 3, 2 July 2010 (2010-07-02), pages 315-324, XP019849402,

## Description

### FIELD OF THE INVENTION

The present invention relates to a method for detecting Episodic falling syndrome (EFS) or prepositions therefore in dogs.

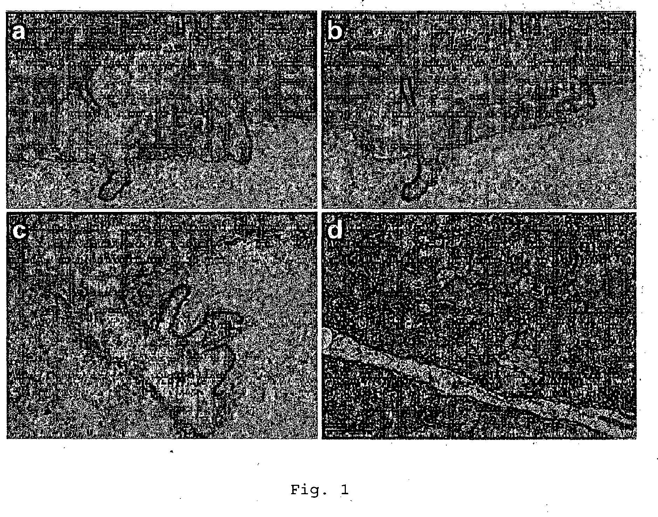

Episodic falling syndrome (EFS) is a well-recognized paroxysmal disorder found in Cavalier King Charles spaniels (CKCS). Episodes begin between fourteen weeks and four years of age and are triggered by exercise, stress, apprehension or excitement (Herrtage and Palmer, 1983). Episodes are of variable frequency and severity but are characterized by progressive hypertonicity involving thoracic and pelvic limbs (Figure 1a) until the dogs are ultimately immobilized in a characteristic 'deer-stalking' or 'praying' position (Figure 1b). Stiffening of all four limbs during exercise can cause falling (Figure 1c), although there is no loss of consciousness or cyanosis. Other clinical signs may include facial muscle stiffness, stumbling, a 'bunny-hopping' gait, arching of the back or vocalization. Curiously, between episodes, dogs appear to be completely normal neurologically. Spontaneous activity was not observed in muscle electrodiagnostic testing, ruling out myotonia congenita (Wright et al. 1986, 1987). Muscle biopsies are typically normal at the light microscopic level, excluding many congenital myopathies. However, EFS has been linked to ultrastructural defects in skeletal muscle including dilatation and proliferation of the sarcoplasmic reticulum, mitochondrial swelling and degeneration (Wright et al. 1986, 1987). EFS has also been compared (Rusbridge, 2005) with startle disease/hyperekplexia, typically characterized by noiseor touch-evoked neonatal hypertonicity due to defects in inhibitory glycine receptor (*GLRA1*, *GLRB;* Shiang et al. 1993; Rees et al. 2002) or glycine transporter GlyT2 (*SLC6A5*) genes (Rees et al. 2006; Harvey et al. 2008). However, a microdeletion in the GlyT2 gene in Irish Wolfhounds results in severe neonatal muscle stiffness and tremor in response to handling (Gill et al. 2011), which is inconsistent with the observed clinical signs of EFS. Comparisons with startle disease may have been made because affected dogs often respond well to the benzodiazepine clonazepam (Garosi et al. 2002), an effective anticonvulsant, anxiolytic and muscle relaxant that is the most effective known treatment for human hyperekplexia (Thomas et al. 2011). However, the carbonic anhydrase inhibitor acetazolamide, used to treat certain types of human episodic ataxia (Tomlinson et al. 2009) and hyperkalemic periodic paralysis (Matthews & Hanna 2010), also appears to have therapeutic value in the treatment of EFS (http://www.cavalierhealth.org/episodic_falling).

Since a ten-year breeder-led investigation into the inheritance of EFS suggested an autosomal recessive mode of inheritance (http://cavalierepisodicfalling.com/), we used a genome-wide association strategy (Karlsson et al. 2007) to map the EFS locus to a defined region of canine chromosome 7. Candidate gene analysis enabled us to identify a microdeletion affecting the brevican gene (*BCAN*), confirm the deletion breakpoint and develop rapid genotyping tests for EFS.

It is, therefore, an object of the present invention to provide an analysis of a genotype of a dog in respect of episodic falling syndrome by a genetic test. By such a genetic test the genetic disposition of a dog can be analysed.

This aim is achieved by the inventions as claimed in the independent claims. Advantageous embodiments are described in the dependent claims.

Even if no multiple back-referenced claims are drawn, all reasonable combinations of the features in the claims shall be disclosed.

This aim is achieved by a genetic test for the identification of the presence or absence of a 15.7 kilo bases deletion in the brevican gene (*BCAN*).

The term 'deletion', when referring to a nucleic acid sequence, has the usual meaning in genetics of an allele in which one or more bases are missing compared to a reference or wild-type sequence. Deletions may be as short as one base-pair. Deletions detected in the present invention may be longer. A deletion in a gene may not only comprise a sequence from the gene itself as it is the case of a deletion within a gene. A deletion in a gene may also extend to sequences next to the gene. For example the deletion may comprise the beginning of the gene and extends further upstream. As a result the whole beginning of the gene is missing together with the sequences upstream. The gene is truncated. Such a deletion is also referred to as a deletion of a specific gene, since this gene is affected by the deletion. Furthermore the region upstream a gene usually comprises important regulatory elements of the gene.

In another embodiment of the invention the deletion is a microdeletion. A microdeletion is the loss of a tiny piece of a chromosome, a piece so small its absence is not apparent on ordinary examination (using a regular light microscope to look at chromosomes prepared in the usual fashion). The detection of microdeletions requires usually special techniques such as high-resolution chromosome banding, molecular chromosome analysis (with FISH), or DNA analysis. Therefore such a microdeletion is of a size of only several kilobases, in particular of 15.7 kb (kilobases).

In such an analysis the presence of the 15.7 kilo bases deletion indicates the genetic disposition for episodic falling syndrome (EFS).

The presence or absence of the 15.7 kilo bases deletion may be detected in various ways. For example the sequence without the deletion or the sequence comprising the deletion may be detected. Especially if the deletion is inherited in an autosomal recessive mode a quantitative analysis may reveal not only affected and not affected dogs, but also carriers of the deletion only of one chromosome by a reduced detection of the analyzed sequence.

The most common method for the detection of the presence or absence of the 15.7 kilo bases deletion is PCR. The presence or absence of an amplified product can be related to the presence or absence of the deletion.

For a quantitative analysis, modern methods like quantitative PCR or multiplex ligation dependent probe amplification (MLPA) may be used.

The invention relates to episodic falling syndrome. There are diseases with similar clinical presentations as episodic falling syndrome. In case of human disorders this is for example exercise-induced dyskinesias. Typically these are nervous disorders of mammals.

In another embodiment of the invention the deletion comprises at least one of the following elements of *BCAN:* regulatory sequences, exon 1, exon 2 and/or exon 3. Exon 1 as used in this specification refers to the 5' UTR region.

The regulatory sequences are promoters and binding sites or other regulatory sequences of the *BCAN* gene, which are located upstream of exon 1.

In another embodiment of the invention the deletion comprises at least exon 1, exon 2 and exon 3.

In another embodiment of the invention the deletion comprises at least exon 1, exon 2 and exon 3, but not exon 4 of the *BCAN* gene. The deletion therefore only comprises a part of the gene including the first three exons.

In another embodiment of the invention the deletion comprises at least one of the sequences according to Seq-ID No. 14, Seq-ID No. 15 and/or Seq-ID No. 16, preferably Seq-ID No. 11, Seq-ID No. 8, Seq-ID No. 9 and/or Seq-ID No. 12.

In another embodiment the deletion comprises a sequence according to Seq-ID No. 13. This sequence comprises the beginning of the *BCAN* gene including the first three exons as well as sequences upstream of the BCAN gene.

In another embodiment the deletion consists of a sequence according to Seq-ID No. 13.

In another embodiment of the invention the deletion is of the size of 15722 bases.

In another embodiment of the invention the deletion starts 1.56 kb downstream of *HAPLN2* and finishes 85 bp downstream of *BCAN* exon 3.

In combination with the deletion the sequence deleted may be exchanged by another sequence. A new sequence may be inserted instead of the deleted sequence. Such a sequence may be several base pairs long. In a preferred embodiment of the invention the sequence shows a microhomology of 1 to 7 bp in a 30 bp region encompassing the breakpoints. A microhomology is the same short sequence of bases in different genes or at different positions within a gene. In another embodiment of the invention this is a 6 bp sequence preferably with the sequence GGCCTT.

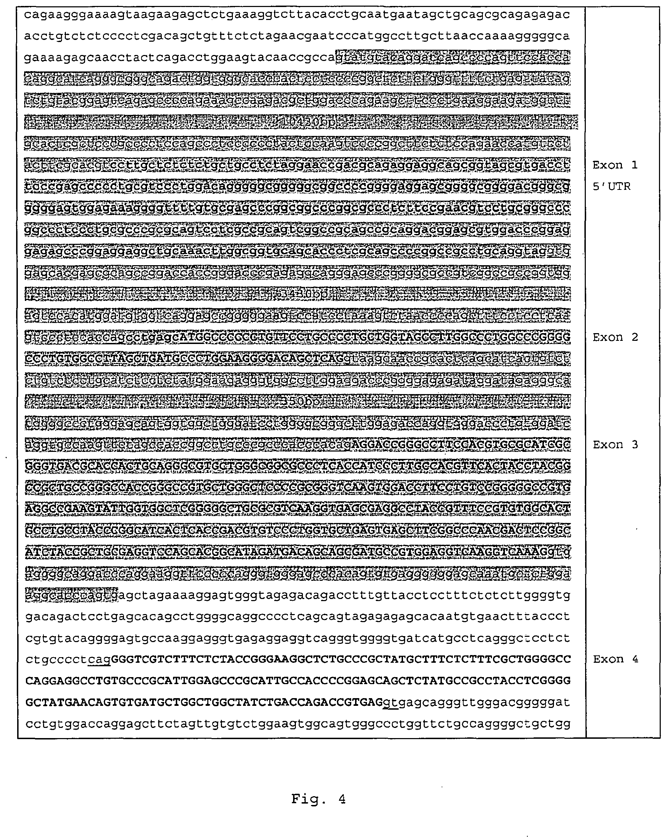

In another embodiment of the invention the deletion leads to a sequence according to Seq-ID No. 1 or Seq-ID No. 2. In this case a short sequence is inserted together with the deletion. The details are shown in figure 4d.

In another embodiment of the invention the presence or absence of the deletion is detected by the detection of the presence or absence of either the sequence without or with the deletion, e.g. Seq-ID No. 1 or Seq-Id No. 2.

In another embodiment of the invention this detection is achieved by the detection of the presence or absence of any of the Seq-ID No. 14, Seq-ID No. 15 and/or Seq-ID No. 16, preferably Seq-ID No. 11, Seq-ID No. 8, Seq-ID No. 9 and/or Seq-ID No. 12, more preferably Seq-ID No. 13. The detection may also be achieved by the detection of a specific part of these sequences (specific sequence). As a positive control the presence or absence of exon 4, preferably sequence Seq-ID No. 17, or any other gene also present in the probe may be used as internal control also for quantitative methods.

In another embodiment of the invention the dog is a canis lupus familiaris. In a preferred embodiment of the invention the dog is a Cavalier King Charles spaniel. In this case the brevican gene and the 15.7 kilo bases deletion are located on chromosome 7.

The invention relates further to a method for determination of the genetic disposition for episodic falling syndrome (EFS) or similar nervous disorders in a dog wherein a 15.7 kilo bases deletion in the brevican gene (*BCAN*) is detected in a DNA sample.

In what follows, individual steps of a method will be described in more detail. The steps do not necessarily have to be performed in the order given in the text. Also, further steps not explicitly stated may be part of the method.

The DNA sample can be any sample from a dog from which genetic information can be retrieved. This can be body fluids like blood, urine or plasma as well as other probes like hair, skin, buccal cells.

The detection of the 15.7 kilo bases deletion may be achieved by various ways. Such techniques which preferably are based on PCR are known to the person skilled in the art. In principle the presence or absence of a sequence or a part of the sequence, which is specific for the presence or absence of the deletion, is analyzed. In case of dogs the deletion may be present on one chromosome but not on the other. In this case the method may reveal three possible results. The specific sequence may be present on both chromosomes, only on one chromosome or on none of the two chromosomes. Depending on the method used for the detection this may result in the presence or absence of different PCR products, like in multiplex PCR, or in a different quantity of a produced PCR product, as in quantitative PCR or multiplex ligation-dependent probe amplification.

Depending on the specific sequence chosen the presence or absence of the sequence can be connected with the genetic disposition of the dog being affected by the disorder, being a carrier or being not affected by the disorder.

A part of a sequence is regarded as specific part of a sequence if its detection allows a judgment if the tested sequence is present or absent in the probe. It is therefore not necessary that this specific sequence is completely unique for all organisms. It is only necessary that the specific part of the sequence is unique for the dog under examination and/or under the conditions of the detection method. Methods to derive such specific parts of a sequence depending of the detection method used are known to the person skilled in the art.

Such a specific part of a sequence may be any sequence from which the presence of the parent sequence may be deduced. These are typically sequences with the length of 20, 30, 40 or 50 base pairs or more. The person skilled in the art knows methods to design probes for the detection of such a sequence. The design of the probes also depends on the detection method. Typically these probes are able to hybridize in a specific manner with the specific sequence chosen. Such probes are usually oligonucleotides. Depending on the detection method these probes can be modified, e.g. with labels like fluorescent dyes or radioactive labels. The probes themselves may comprise modified nucleotides or a modified backbone. For PCR a pair of primers has to be designed, which are specific under PCR conditions. For other methods like qPCR or MLPA the probes may consist of one or more oligonucleotides, e.g. two oligonucleotides, which only in combination lead to a positive test. The detection of the presence of the specific part of the sequence is then related to the absence or presence of the deletion. Examples for the oligonucleotide probes or primers can be found in the examples of the present application. It is therefore not necessary to detect the complete sequence. The specific part of the sequence is sufficient.

The term 'primer' refers to a single-stranded oligonucleotide capable of acting as a point of initiation of template-directed DNA synthesis under appropriate conditions, in an appropriate buffer and at a suitable temperature. The appropriate length of a primer depends on the intended use of the primer but typically ranges from 15 to 30 nucleotides. A primer sequence need not be exactly complementary to a template but must be sufficiently complementary to hybridize with a template. The term 'primer pair' means a set of primers including a 5' upstream primer, which hybridizes to the 5' end of the DNA sequence to be amplified and a 3' downstream primer, which hybridizes to the complement of the 3' end of the sequence to be amplified.

In another embodiment of the invention the presence or absence of the deletion is detected by the detection of a sequence comprising Seq-ID No. 13 or a specific part of Seq-ID No. 13. The detection of the sequence also may be achieved by a PCR-product comprising this sequence or a part of this sequence, e.g. if only one of the primers hybridizes with the sequence to be detected. The presence of the sequence is related to the absence of the deletion.

In another embodiment of the invention the presence or absence of the deletion is detected by the detection of at least one sequence comprising the sequence or a specific part of the sequence selected from the group comprising regulatory sequences, exon 1, exon 2 and/or exon 3 of the *BCAN* gene. In a preferred embodiment the presence or absence of the deletion is detected by the detection of at least one sequence comprising the sequence or a specific part of the sequence selected from the group comprising exon 1, exon 2 and/or exon 3 of the *BCAN* gene.

In another embodiment of the invention the presence or absence of the deletion is detected by the detection of at least one sequence comprising the sequence or a specific part of the sequence selected from the group comprising regulatory sequences according to Seq-ID No. 11, Seq-ID No. 8, Seq-ID No. 9 and/or Seq-ID No. 12.

In another embodiment of the invention the presence or absence of the deletion is detected by the detection of at least one sequence comprising the sequence or a specific part of the sequence selected from the group comprising regulatory sequences according to Seq-ID No. 11, exon 1 according to Seq-ID No. 14, exon 2 according to Seq-ID No. 15 and/or exon 3 according to Seq-ID No. 16. The presence of at least one of these sequences detected hereby indicates the absence of the deletion.

The detection of the sequence also may be achieved by a PCR-product comprising the sequence or a part of the sequence, e.g. if only one of the primers hybridizes within the sequence. The presence of all of the detected sequences indicates the absence of the deletion. Usually the examination of one specific sequence is sufficient.

In another embodiment of the invention the detection of the deletion is achieved by the detection of the presence or absence of at least one of the *BCAN* regulatory sequences, exon 1, exon 2 and/or exon 3 of the *BCAN* gene or a specific sequence thereof. The presence of the sequences hereby indicates the absence of the deletion.

In another embodiment of the invention the detection of the deletion is achieved by the detection of the presence or absence of at least one of exon 1, exon 2 and/or exon 3 of the *BCAN* gene or a specific sequence thereof. The presence of the sequences hereby indicates the absence of the deletion.

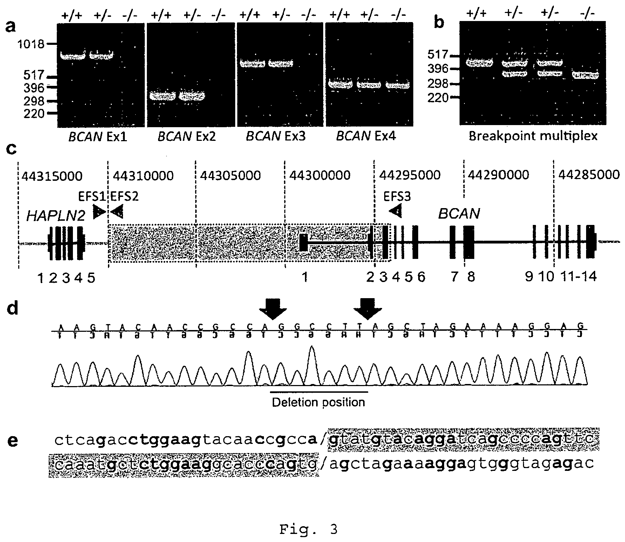

In another embodiment of the invention the detection of the deletion is achieved the detection of a sequence specific for the absence of the deletion as well as the detection of a sequence specific for the presence of the deletion. This may be achieved by multiplex PCR using primer sets resulting in PCR products of specific length for each case (absence or presence of the deletion). Usually PCR products share one primer, which hybridizes with a part of the gene not affected by the deletion. The other primers hybridize within the deletion in order to detect the presence of the deletion. The other primer may hybridize with the sequence created by the deletion or a part of the gene after the deletion (in respect of the other primer). In such an essay affected, carrier and not affected genotypes may be detected due to the pattern of PCR products. An example for such an assay is shown in figure 3b.

In another embodiment of the invention the detection of the deletion is achieved by the quantification of the presence of at least one sequence comprising the sequence or a specific part of the sequence selected from the group comprising the *BCAN* regulatory sequences, exon 1, exon 2 and/or exon 3, preferably exon 1, exon 2 and/or exon 3. Typically the quantification is achieved by the comparison to quantification of the presence of a different sequence within the same sample, for example exon 4 or another gene, which is not influenced by the deletion.

Such quantification is usually achieved with methods based on quantitative PCR or real-time PCR. In these methods the amount of product of a PCR reaction is measured. From this the amount of the PCR target (the sequence to be detected) can be calculated. Depending on the method this target does not need to be the gene itself. In MLPA, the ligated product of two hybridized probes is amplified. The amount of target is then related to the amount of this ligated product.

In another embodiment of the invention the detection of the deletion is achieved by the quantification of the presence of at least one sequence comprising the sequence or a specific part of the sequence selected from the group comprising Seq-ID No. 11, Seq-ID No. 8, Seq-ID No. 9 and/or Seq-ID No. 12, preferably Seq-ID No. 14, Seq-Id No. 15 and/or Seq-ID No. 16. Typically the quantification is achieved by the comparison to an amplification product from a different sequence within the same sample, for example exon 4 or another gene, which is not influenced by the deletion.

In another embodiment of the invention the detection of the deletion is achieved by detection of a sequence or a specific part of a sequence according to Seq-ID No. 1 or Seq-ID No. 2.

The absence of the sequence indicates the absence of the deletion.

In another embodiment of the invention the dog is a Cavalier King Charles spaniel. In this case the detection is carried out on chromosome 7, where the *BCAN* gene resides.

The invention also relates to an isolated nucleic acid sequence comprising a sequence according to Seq-ID No. 1 or 2.

The invention also relates to an isolated nucleic acid sequence, which encodes the *BCAN* gene and comprises the 15.7 kilo bases deletion comprising at least one of regulatory sequences, exon 1, exon 2 and exon 3.

The invention also relates to a brevican (*BCAN*) gene, which comprises a sequence according to Seq-ID No. 1 or 2. As shown in figure 3c and 3d the deletion comprises the beginning of the gene. The first 14 bases of Seq-ID No. 1 or the last 14 bases of Seq-ID No. 2 were located upstream of the *BCAN* gene before the deletion. By the deletion this sequence is regarded as part of the truncated *BCAN* gene.

In another embodiment of the invention the *BCAN* gene is the brevican gene of a Cavalier King Charles spaniel. Thus, it is located on chromosome 7. The deletion is located within the interval 44310000 and 44290000 on this chromosome, preferably the deletion starts 1.56 kb downstream of *HAPLN2* and finishes 85 bp downstream of *BCAN* exon 3.

A 'nucleic acid', 'polynucleotide' or 'oligonucleotide' is a polymeric form of nucleotides of any length, may be DNA or RNA, and may be single- or double-stranded. Nucleic acids may include promoters or other regulatory sequences. Oligonucleotides are usually prepared by synthetic means. A reference to the sequence of one strand of a double-stranded nucleic acid defines the complementary sequence and except where otherwise clear from context, a reference to one strand of a nucleic acid also refers to its complement.

An 'isolated' nucleic acid means a nucleic acid species that is the predominant species present in a composition. Isolated means the nucleic acid is separated from at least one compound with which it is associated in nature. A purified nucleic acid comprises (on a molar basis) at least about 50, 80 or 90 percent of all macromolecular species present.

The invention further relates to the use of the described analysis or method for the selection for breeding of dogs, preferably Cavalier King Charles spaniels. This genetic test allows especially the identification of carriers, which are not distinguishable from normal dogs by other methods.

### Brief summary of the invention

Episodic falling syndrome (EFS) is a canine paroxysmal hypertonicity disorder found in Cavalier King Charles spaniels. Episodes are triggered by exercise, stress or excitement and characterized by progressive hypertonicity throughout the thoracic and pelvic limbs, resulting in a characteristic 'deer-stalking' position and/or collapse. We used a genome wide association strategy to map the EFS locus to a 3.48 Mb critical interval on canine chromosome 7. By prioritizing candidate genes on the basis of biological plausibility, we found that a 15.7 kb deletion in *BCAN,* encoding the brain-specific extracellular matrix proteoglycan brevican, is associated with EFS. This represents a compelling causal mutation for EFS, since brevican has an essential role in the formation of perineuronal nets governing synapse stability and nerve conduction velocity. Mapping of the deletion breakpoint enabled the development of Multiplex PCR and Multiplex Ligation-dependent Probe Amplification (MLPA) genotyping tests that can accurately distinguish normal, carrier and affected animals. Wider testing of a larger population of CKCS dogs without a history of EFS from the USA revealed that carriers are extremely common (12.9%). The development of molecular genetic tests for the EFS microdeletion will allow the implementation of directed breeding programs aimed at minimizing the number of animals with EFS and enable confirmatory diagnosis and pharmacotherapy of affected dogs.

### Discussion

The genomic architecture of pure-bred dog lines is ideal for the identification of loci responsible for autosomal recessive traits using genome-wide association mapping (Karlsson et al. 2007, Drögemüller et al. 2009). In this study, we demonstrate that this technique can be used successfully on minimal samples sets, since we located the EFS locus using DNA samples from only five affected and seven breed-matched control dogs. Since a homozygous haplotype spanning 6.35 Mb was identified in affected animals, it is questionable whether further SNP typing would have generated additional useful data. In fact, a single recombination event in an obligate carrier allowed us to narrow the critical interval to 3.48 Mb. This region contained >100 genes, including ligand-gated ion channels, K+ channels, transporters, mitochondrial proteins and several genes known to be involved in neurological disorders in humans. For example, mutations in *CHRNB2* are associated with nocturnal frontal lobe epilepsy (De Fusco et al. 2000) and *TPM3* mutations are associated with nemaline myopathy (Laing et al. 1995). However, many of these genes were rapidly eliminated as candidates due to either: i) poor correlation of EFS clinical signs with the equivalent human disorders or ii) systematic resequencing of the genes. Consistent with the unique clinical signs observed in affected dogs, we discovered that a homozygous microdeletion affecting *BCAN* is associated with EFS in CKCS dogs, confirming that this disorder is inherited in an autosomal recessive manner. This mutation was not detected in control DNA samples from 53 other dog breeds, confirming the unique nature of this genomic rearrangement.

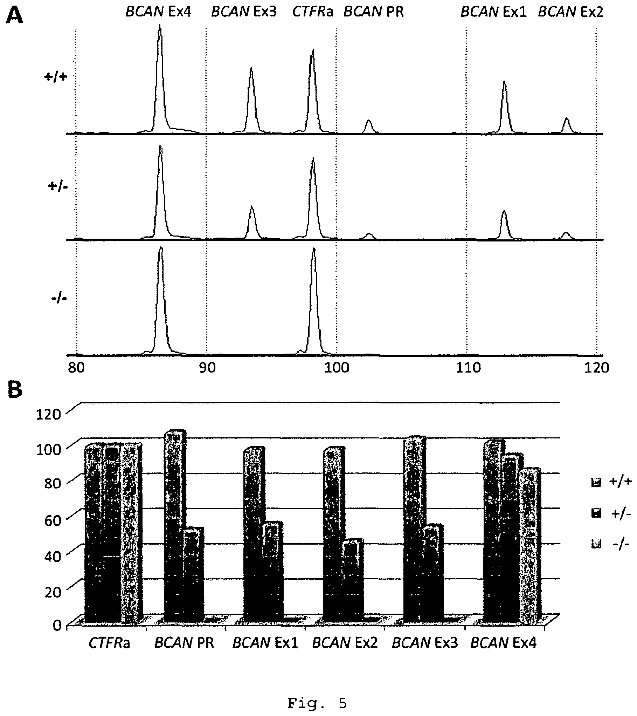

Brevican belongs to the lectican family of aggregating extracellular matrix (ECM) proteoglycans, which comprises aggrecan, brevican, neurocan and versican. Although mutations in the aggrecan and versican genes (*ACAN*: 15q26.1 and *VCAN:* 5q14.2-14.3) have been linked to different connective tissue disorders (Gleghorn et al. 2005; Stattin et al. 2010; Miyamoto et al. 2005; Fig. 5), no mutations in the brevican or neurocan genes (*BCAN:* 1q23.1 and *NCAN:* 19p13.11) have been identified to date. Brevican and neurocan are highly expressed in the central nervous system, where they are found in specialized extracellular matrix structures called perineuronal nets that play a role in cell adhesion, migration, axon guidance and neuronal plasticity (Brakebusch et al. 2002). Brevican, versican, HAPLN2/BRAL1, tenascin-R and phosphacan are also present at the nodes of Ranvier on large diameter myelinated axons (Bekku et al. 2009, 2010) where cations are accumulated and depleted in the local extracellular nodal region during action potential propagation. The ECM complex at nodes of Ranvier is thought to play a pivotal role in maintaining a local microenvironment, acting as a diffusion barrier for K+ and Na+ around the perinodal extracellular space (Oohashi et al. 2002; Bekku et al. 2010). Thus, disruption of ECM complexes governing synapse stability and nerve conduction velocity are likely to underlie the EFS phenotype. Certainly, since EFS appears to result from a central nervous system rather than a muscle defect, the associated sarcoplasmic reticulum pathology is likely to be a secondary manifestation of muscle overstimulation (Engel, 2004).

Interestingly, *BCAN* knockout mice were not reported (Brakebusch et al. 2002) to have a phenotype similar to EFS - although an increased grip-strength was noted, which could be indicative of increased muscle tone. However, it is questionable whether current mouse phenotyping tests will reveal neurological disorders evoked by strenuous exercise, stress, apprehension or excitement, since these conditions are generally avoided in mouse care. It is also noteworthy that EFS clearly shows a variable age of onset and penetrance in CKCS dogs. In wider population testing, we identified two dogs that were homozygous for the *BCAN* microdeletion that did not show clinical signs of EFS in the presence of their owners. Interestingly, similar findings were reported for the dynamin (DNM1) mutation (p.R256L) underlying exercise-induced collapse in Labrador retrievers (Patterson et al. 2008). It is plausible that these dogs have not been exposed to sufficient exercise or excitement to trigger an EFS episode, or that genetic or environmental modifying factors affect the onset of EFS. It is notable that the two asymptomatic dogs in our study were described as 'lazy' and 'clingy' by their respective owners, suggesting that adaptive behavior may occur in dogs with EFS.

In summary, we have shown that an inactivating microdeletion affecting *BCAN* is associated with EFS in CKCS dogs. Identification of the deletion breakpoint has allowed the development of diagnostic tests that have revealed a high prevalence of carriers (12.9%) in clinically unaffected dogs from the USA. These genetic tests will enable future identification of heterozygous animals (which have no discernable phenotypic difference to wild-type animals) allowing directed breeding programs to be implemented, and confirmatory diagnosis and appropriate pharmacotherapy of affected animals. Since this also represents the first report of a genetic disorder involving a neuronal-specific ECM proteoglycan, we suggest that *BCAN* and *NCAN* should be considered as candidate genes for genetic analysis in unresolved cases of human disorders with similar clinical presentations to EFS, such as paroxysmal exercise-induced dyskinesias) (Weber & Lerche 2009) or episodic ataxias (Tomlinson et al. 2009).

### BRIEF DESCRIPTION OF THE FIGURES AND TABLES

Other objects and advantages of the present invention may be ascertained from a reading of the specification and appended claims in conjunction with the drawings therein.

For a more complete understanding of the present invention; reference is established to the following description made in connection with accompanying drawings in which:
Figure 1. Clinical signs of Episodic Falling Syndrome and musclepathology. A 5- month-old female Cavalier King Charles Spaniel presented with typical episodes of excitement or exercise-induced muscle stiffness (a, hypertonicity) that would involve all four limbs and progress to an usual 'deer stalking' or 'praying' posture (b), eventually resulting in falling (c). While the muscle was normal histologically by light microscopy, electron microscopy revealed that the sarcoplasmic reticulum (sr) appeared dilated and contained finely granular material. Mitochondria (m) and myofibrils were normal in appearance. Scale bar = 0.31 µm.

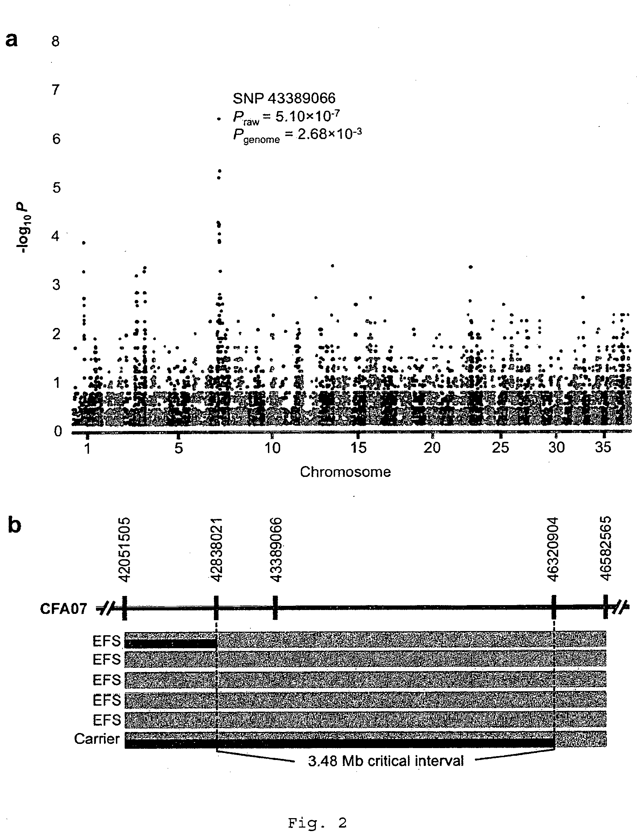

Figure 2. Mapping the Episodic Falling Syndrome locus.
   (a) -log10 of Praw values (Y axis) for genome wide association using five EFS and seven control dogs are plotted for each chromosome (X axis). A single major signal was detected on chromosome 7. (b) A 3.48 Mb critical interval encompassing SNP 43389066 is defined by recombination events in an affected CKCS and an obligate carrier.
Figure 3. (a) PCR panels for *BCAN* exons 1-4 showing that amplicons for the first three exons of the brevican gene can be generated from genomic DNA from normal (+/+) or obligate carrier (+/-) samples, but cannot be amplified from an equivalent EFS sample (-/-). By contrast, *BCAN exon* 4 can be amplified from all genotypes. (b) Multiplex PCRs with primers flanking the 15.7 kb *BCAN* microdeletion allowed simultaneous detection of the wild-type *BCAN* allele (primers EFS1 and EFS2, 393 bp) in normal (+/+) or EFS carrier (+/-) animals, while the EFS allele (primers EFS1 and EFS3, 279bp) is detected in both EFS carrier and affected (-/-) dogs. Note that the two carriers shown have both wild-type and EFS amplicons, as expected for a heterozygous genotype. (c) Schematic diagram showing the genomic organization of the *HAPLN2* and *BCAN,* the position of the deletion (grey shaded rectangle with dotted lines) and EFS1-3 primers. (d) Sequence spanning the *BCAN* deletion breakpoint, showing an additional non-homologous inserted sequence indicated by arrows (upper sequence Seq-ID No. 1; lower sequence Seq-ID No. 2). (e) Alignment of the DNA sequence immediately flanking the deletion breakpoint indicating local microhomology (upper left sequence Seq-ID No. 3; upper right sequence Seq-ID No. 4; lower left sequence Seq-ID No. 5; lower right sequence Seq-ID No. 6).
Figure 4. Position of the EFS microdeletion. Genomic DNA from canine chromosome 7 highlighting exons 1-4 of *BCAN* (shown in bold) and the position of the 15.7 kb EFS microdeletion (grey shading). The first sequence of figure 1 is Seq-ID No. 7 (grey shaded sequence: Seq-ID No. 11). The second sequence comprising exon 1 (Seq-ID No. 14) is Seq-ID No. 8. The third sequence comprising exon 2 (Seq-ID No. 15) is Seq-ID No. 9. The fourth sequence comprising exons 3 (Seq-ID No. 16) and 4 (Seq-ID No. 17) is Seq-ID No. 10. The grey shaded sequence in the fourth sequence is shown in Seq-ID No. 12.
Figure 5. Confirmation of the *BCAN* microdeletion using Multiplex Ligationdependent Probe Amplification. (a) MLPA analysis revealed robust detection of a control probe (CFTRa) and probes for the *BCAN* promoter/regulatory region (PR) and exons 1-4. However, signals for probes PR and exons 1-3 were reduced by 46-55% in heterozygous (+/-) animals and abolished in homozygous animals (-/-), consistent with a loss of probe binding sites in genomic DNA.

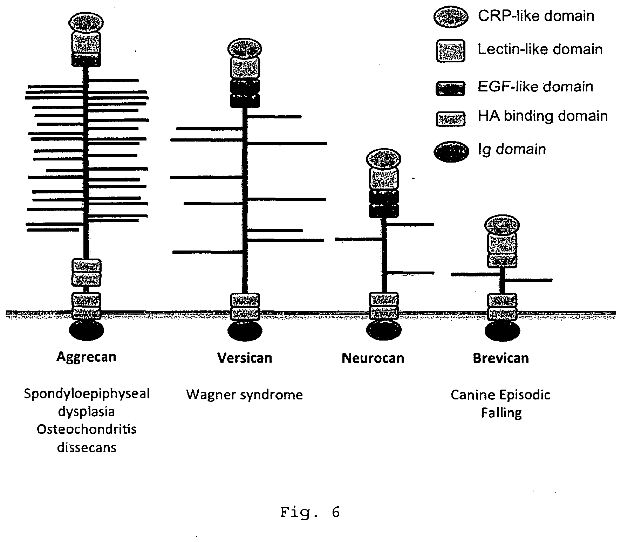

Figure 6. Modular organization of the superfamily of hyaluronan-binding proteins and associated disorders. Mutations in *ACAN,* encoding aggrecan - a major component of cartilage, have been implicated in spondyloepiphyseal dysplasia type Kimberley (Gleghorn et al. 2005) and familial osteochondritis dissecans (Stattin et al. 2010). Mutations in *VCAN,* encoding versican, are associated with Wagner syndrome and erosive vitreoretinopathy, disorders affecting the connective tissue of the eye (Miyamoto et al. 2005). Modified from Maeda et al. 2010.
Table 1. *BCAN* genotypes in CKCS cohorts and other dog breeds
Table 2. Primer sequences for canine *BCAN* and *HAPLN2* exon amplification.
Table 3. Chromosome 7 single nucleotide polymorphisms associated with EFS. A total of 17 SNPs on canine chromosome 7 were associated with EFS (P_{raw} values ≤ 0.0001).
Table 4. Probes for MLPA; Key: P- = 5' phosphate group, **Bold:** 5' PCR primer target, Underlined: 3' PCR primer target, *italic* = stuffer; Tm: melting point; Size: size of the resulting product. Observed migration: *BCAN* Ex4 86 bp; *BCAN* Ex3 94 bp; *CFTRa* control 98 bp; *BCAN* PR 102 bp; *BCAN* Ex1 113 bp; *BCAN* Ex2 118 bp.

Genotypes revealed by multiplex PCRs were determined as described in the methods. Dogs were evaluated on the basis of available clinical data and placed into one of the phenotype categories above. Note that all clinically affected animals were homozygous for the *BCAN* deletion, whilst obligate carriers were heterozygous. As well as wild-type animals and carriers of the *BCAN* deletion, two dogs homozygous for the BCAN deletion, which were not reported to have EFS, were detected in a cohort of animals related to known EFS dogs. Carriers were also detected in CKCS with no history of EFS, but not in control DNA samples from 53 other dog breeds including: Airedale terrier, Akita Basenji, American Staffordshire Terrier, American Cocker Spaniel, American Eskimo Dog, Australian Shepherd, Bernese Mountain Dog, Bluetick Coonhound, Border Collie, Boston Terrier, Boxer, Boykin Spaniel, Briard, Bull Mastiff, Bulldog, Cairn Terrier, Catahoula Leopard Dog, Chihuahua, Collie, Dachshund, Dalmatian, English Setter, English Springer Spaniel, Flat Coated Retriever, German Shepherd, Giant Schnauzer, Golden Retriever, Great Dane, Havanese, Siberian Husky, Irish Setter, Italian Greyhound, Labrador Retriever, Miniature Pinscher, Miniature Poodle, Miniature Schnauzer, New Guinea Singing Dog, Norwegian Elkhound, Petit Basset Griffon Vendeen, Pomeranian, Portuguese Podengo Pequeno, Pug, Pyrenean Shepherd, Schipperke, Shetland Sheepdog, Swedish Vallhund, Tibetan Terrier, Toy Fox Terrier, Weimaraner, Welsh Terrier, West Highland White Terrier, Wire Fox Terrier and Yorkshire Terrier. Where possible, two unrelated dogs were tested for each breed.

### Materials and Methods

Light and electron microscopy: For light microscopy, unfixed biopsies from the biceps femoris, vastus lateralis and triceps brachii muscles were collected from five affected CKCS dogs under general anesthesia and frozen in isopentane pre-cooled in liquid nitrogen. Cryosections were cut (8 µm) and the following histochemical stains and reactions performed: hematoxylin and eosin, modified Gomori trichrome, periodic acid Schiff, phosphorylase, esterase, ATPase reactions at pH of 9.8 and 4.3, nicotinamide adenine dinucleotide-tetrazolium reductase, succinic dehydrogenase, acid phosphatase, alkaline phosphatase and oil red O. For electron microscopy, glutaraldehyde-fixed muscle specimens were post-fixed in osmium tetroxide, and dehydrated in serial alcohol solutions and propylene oxide prior to embedding in araldite resin. Thick sections (1 pm) were stained with toludine blue for light microscopy and ultrathin sections (60-90 nm) were stained with uranyl acetate and lead citrate for electron microscopy.

### Genomic DNA preparation:

Whole blood or buccal cells were collected from EFS affected, clinically normal relatives of affected, and clinically normal unrelated control CKCS dogs. Genomic DNA was isolated using the Gentra Puregene Blood Kit (QIAGEN, Valencia, USA). Additional DNA samples isolated from whole blood or buccal cells from other pure bred-dogs were available from unrelated studies and other sources.

### Genome-wide association mapping:

Thirteen CKCS genomic DNA samples isolated from blood (five cases, one obligate carrier and seven controls) were genotyped for 127,000 SNPs on the Affymetrix CanineSNP Array version 2 (http://www.broadinstitute.org/mammals/dog/caninearrayfaq.html ). Arrays were processed at the Clemson University Genomics Institute (http://www.genome.clemson.edu/) using the GeneChip human mapping 250K Sty assay kit (Affymetrix, Santa Clara, USA). The GeneChip human mapping 500K assay protocol was followed, but with a hybridization volume of 125 µl (Karlsson et al. 2007). Raw CEL files were genotyped using Affymetrix Power Tools software. SNPs having >10% missing data and =60% heterozygosity were removed. Data for 58,873 SNPs were formatted for PLINK (Purcell et al. 2007) and case/control analyses with 100,000 permutations were performed for five cases and seven controls (the obligate carrier was excluded from analysis).

### PCR and DNA sequencing:

PCR primers were designed to amplify exons and flanking splice donor, acceptor and branch-point sites, from gene structures derived in silico using the UCSC Genome Browser. For exon-specific primers for *BCAN* and *HAPLN2* exon amplification see Table 2. PCR was performed using 50 ng genomic DNA as template and AccuPrime Pfx SuperMix supplemented with betaine for 40 cycles of 94°C for 1 min, 60°C for 1 min, 68°C for 1 min. PCR products were gel purified using a QiaQuick gel extraction kit (QIAGEN, Crawley, UK) for TOPO cloning (pCR4Blunt-TOPO; Invitrogen, Renfrew, UK). Sanger DNA sequencing was performed, by DNA Sequencing & Services (MRCPPU, College of Life Sciences, University of Dundee, Scotland) using Applied Biosystems Big-Dye version 3.1 chemistry on an ABI 3730 automated capillary DNA sequencer, DNA sequences were analyzed using Sequencher 4.10 (Gene Codes Corporation, Ann Arbor,USA). For multiplex PCRs, the diagnostic primer set is: EFS1 5'- aaggtcttacacctgcaatgaatag-3'(Seq-ID No. 18), EFS2 5'- agcaaatgtaaagtcctgtgaccat-3' (Seq-ID No. 19) and EFS3 5'- agttcacattgtgctctctctactg-3' (Seq-ID No. 20).

### Multiplex ligation-dependent probe amplification (MLPA) analysis:

Five MLPA probe sets were designed corresponding to the promoter region (PR) and exons 1 (5' UTR), 2, 3 and 4 of the canine brevican gene (*BCAN;* NC-006589 on chromosome 7; Table 4). Criteria for MLPA probe design were as previously described (Schouten et al. 2002). A control probe pair was designed to recognize an unrelated gene (*CFTR:* NC-006596 on chromosome 14). Probes generated amplification products ranging in size from 88 to 115 bp and had annealing temperatures higher than 70°C as recommended in RAW Probe software (MRC-Holland, Amsterdam, The Netherlands) using standard MLPA conditions (Schouten et al. 2002). PCR products were analyzed on an ABI 3130XL capillary electrophoresis apparatus (Applied Biosystems, Lennik, Belgium). Normalization of *BCAN* specific probe signals was performed by dividing the values obtained by the combined signal of the control probes.

### Results

### Light and electron microscopy:

Unfixed cryosections of muscle biopsies were histologically normal at the light microscopic level with no abnormalities detected following any of the histochemical stains and enzyme reactions employed. Contrary to previous reports (Wright et al. 1986, 1987) electron microscopy revealed normal myofibrillar and mitochondrial morphology, although swelling of the sarcoplasmic reticulum was confirmed (Fig. 1d).

### Genome-wide association mapping and candidate gene resequencing:

A total of 17 single nucleotide polymorphisms (SNPs) were associated with EFS (Praw values = 0.0001) (Table 3). The most significant result was for SNP 43389066 (Praw = 5.10x10-7, Pgenome = 2.68x10-3) (Fig. 2a). All significant SNPs were located within a 7.2 Mb region on canine chromosome 7. A critical interval of 3.48 Mb (from 7.42838021 to 7.46320904) was delimited by recombinant chromosomes identified in one EFS dog and an obligate carrier (Fig. 2b). In order to identify the mutation associated with EFS, we prioritized several genes for resequencing based on biological plausibility. These encoded ligand-gated or voltage-gated ion channels (*CHRNB2, HCN3, KCNN3*), mitochondrial (*MRPL24, MTSO1, MTX1, SLC25A44*), muscle (*MEF2D, TPM3*) or brain-expressed proteins (*ARHGEF11, BCAN, GBA, HAPLN2, NES, RIT1, SYT11, UBQLN4*)*.* Curiously, amplification of *BCAN* exons 1, 2 and 3 consistently failed with multiple primer sets when using genomic DNA from affected animals, while DNAs from carriers and unaffected dogs amplified reliably (Fig. 3a). Because no preferential amplification was observed for the adjacent gene, *HAPLN2* - encoding hyaluronan and proteoglycan link protein 2/Bral1 (Hirakawa et al. 2000; Oohashi et al.2002; Bekku et al. 2010) - we suspected that a microdeletion affecting *BCAN* regulatory sequences and exons 1-3 was associated with EFS.

### Deletion breakpoint identification and development of diagnostic tests:

Further primer walking experiments enabled us to clone and sequence a DNA fragment containing the deletion breakpoint and develop a multiplex PCR assay that distinguishes between affected, carrier and normal dogs (Fig. 3b). Sequence analysis of the breakpoint amplicon revealed a 15.7 kb microdeletion starting 1.56 kb downstream of *HAPLN2,* encompassing *BCAN* promoter elements and exons 1 (5' untranslated), 2 and 3, finishing 85 bp downstream of *BCAN* exon 3 (Fig. 3c). The microdeletion amplicon also contained a 6 bp inserted sequence (GGCCTT; Fig. 3d) typical of deletions resulting from non-homologous end joining (NHEJ) or microhomology-mediated end joining (MMEJ). Several regions of microhomology (1-7 bp) were identified in a 30 bp region encompassing the breakpoints (Fig. 3e). Interestingly, 5 of 6 bp of the reverse complemented inserted sequence aligns to the largest region of microhomology. We also noted an abundance of short interspersed element (SINE) insertions at the 5' end of the deleted sequence, which could cause the formation of secondary structures that facilitate chromosomal rearrangement (Chuzhanova et al. 2003). We also detected the presence of the *BCAN* microdeletion (Fig. 4) using MLPA (Schouten et al. 2002) and canine-specific probe sequences (Table 4). This quantitative analysis also confirmed that EFS is associated with a loss of *BCAN* promoter/regulatory elements and exons 1-3 in heterozygous carriers and homozygous affected animals (Fig. 5).

### Rapid genotyping using multiplex genotyping in different dog populations:

To assess the prevalence of the EFS microdeletion, we used our multiplex PCR assay to test several CKCS populations and other dog breeds (Table 1). All EFS study dogs from the UK (n = 2), USA (n = 6) and New Zealand (n = 2) were homozygous for the microdeletion, and all obligate carriers were heterozygous (n = 8, dogs from USA and New Zealand). In animals related to affected dogs, we found 9 normal animals and 10 carriers. Interestingly, in this group, two dogs without a clinical history of EFS were homozygous for the microdeletion. Lastly, in dogs with no known clinical history of EFS sourced from the USA, the carrier frequency was 12.9% (20/155) suggesting that the EFS microdeletion is present at a high frequency in this population. Notably, the mutation was not detected in multiplex PCRs conducted on control DNA samples from 53 other breeds of dog (Table 1).

While the present inventions have been described and illustrated in conjunction with a number of specific embodiments, those skilled in the art will appreciate that variations and modifications may be made without departing from the principles of the inventions as herein illustrated, as described and claimed. The present inventions may be embodied in other specific forms without departing from their spirit or essential characteristics. The described embodiments are considered in all respects to be illustrative and not restrictive. The scope of the inventions is, therefore, indicated by the appended claims, rather than by the foregoing description. All changes which come within the meaning and range of equivalence of the claims are to be embraced within their scope.

**Table 1**

| Phenotype | Normal | Carrier | Affected |
|---|---|---|---|
| CKCS EFS affected | 0/10 | 0/10 | 10/10 |
| Study CKCS EFS carrier | 0/8 | 8/8 | 0/8 |
| Study CKCS related to affected or carrier | 9/21 | 10/21 | 2/21 |
| CKCS with no EFS history | 135/155 | 20/155 | 0/155 |
| 53 dog breeds with no EFS history | 93/93 | 0/93 | 0/93 |

**Table 2**

| Gene | Exon | Forward primer | Reverse primer |
|---|---|---|---|
| *BCAN* | 1 | cttctctccagaaccatgtcctac Seq-ID No. 21 | acacagcaagtaagtggcagagtt Seq-ID No. 22 |
| | 2 | gagttaacggtgaggttgggacagt Seq-ID No. 23 | cccacagtgccctctatcctatctc Seq-ID No. 24 |
| | 3 | aagatttggggacagatctggagag Seq-ID No. 25 | ccccaagagagaaaggaggtaacaa Seq-ID No. 26 |
| | 4 | ctttctctcttggggtggacagact Seq-ID No. 27 | ggtcctggatggttgacctgag Seq-ID No. 28 |
| | 5 | gggtgtctctgcagaagaaaacaat Seq-ID No. 29 | gaagacctctggacagcaccg Seq-ID No. 30 |
| | 6 | ggcccagagaagccagccta Seq-ID No. 31 | gggaaacttcagagctcaagtctgt Seq-ID No. 32 |
| | 7 | ccacagggaagatgagtgagaattg Seq-ID No. 33 | ccagcatcactctggacacctt Seq-ID No. 34 |
| | 8 | ctcaccctccacagcccctt Seq-ID No. 35 | ccagagatcatgtgacccagagctt Seq-ID No. 36 |
| | 9 | gtgatagctcccaagacaaggagat Seq-ID No. 37 | gcagggtccaggcttcaggtcta Seq-ID No. 38 |
| | 10 | ctctgctggctctctggcat Seq-ID No. 39 | tgagtgggagagagcaggtga Seq-ID No. 40 |
| | 11 | acggacagggaagcagggaa Seq-ID No. 41 | gcggaggcagaagtgcttgg Seq-ID No. 42 |
| | 12 | tgggcctccactcctcatcc Seq-ID No. 43 | ttacacacatgcggcttgggtc Seq-ID No. 44 |
| | 13 | atcccgggtctccaggatca Seq-ID No. 45 | gagcccagggctactgttggata Seq-ID No. 46 |
| | 14 | ccagggctagtgtttggatgagat Seq-ID No. 47 | ccctcacgtggtcacttcctatg Seq-ID No. 48 |
| *HAPLN2* | 1-2 | gacattccccacacacaccaag Seq-ID No. 49 | cgcctgtttcgcttcatagtaattg Seq-ID No. 50 |
| | 3-5 | tgtctgccggctctccctaa Seq-ID No. 51 | aggatacagacccatcctgaagcac Seq-ID No. 52 |

**Table 3**

| SNP | P-value |
|---|---|
| 7.43389066 | 5.10×10⁻⁷ |
| 7.46204875 | 5.61×10⁻⁶ |
| 7.46283892 | 5.61×10⁻⁶ |
| 7.42051505 | 7.65×10⁻⁶ |
| 7.39115202 | 6.12×10⁻⁵ |
| 7.39142101 | 6.12×10⁻⁵ |
| 7.43140878 | 6.73×10⁻⁵ |
| 7.43197311 | 6.73×10⁻⁵ |
| 7.43230030 | 6.73×10⁻⁵ |
| 7.43298329 | 6.73×10⁻⁵ |
| 7.43302986 | 6.73×10⁻⁵ |
| 7.43867010 | 6.73×10⁻⁵ |
| 7.43917092 | 6.73×10⁻⁵ |
| 7.44014865 | 6.73×10⁻⁵ |
| 7.44059025 | 6.73×10⁻⁵ |
| 7.41416845 | 7.19×10⁻⁵ |
| 7.42027729 | 7.19×10⁻⁵ |

**Table 4**

| Probe | Sequence | Tm °C | %GC | Size |
|---|---|---|---|---|
| *CFTRa* | | 72 | 48 | 100 |
| | | 75 | 48 | |
| *BCAN* PR | | 72 | 60 | 104 |
| | | 72 | 65 | |
| *BCAN* Exon 1 | | 70 | 62 | 111 |
| | | 71 | 68 | |
| *BCAN* Exon 2 | | 72 | 59 | 115 |
| | | 70 | 59 | |
| *BCAN* Exon 3 | | 77 | 64 | 96 |
| | | 72 | 57 | |
| *BCAN* Exon 4 | | 72 | 54 | 88 |
| | | 71 | 59 | |

### References Cited

Bekku Y, Rauch U, Ninomiya Y, Oohashi T. Brevican distinctively assembles extracellular components at the large diameter nodes of Ranvier in the CNS. J Neurochem 2009; 108:1266-1276.
Bekku Y, Vargová L, Goto Y, Vorisek I, Dmytrenko L, Narasaki M et al. Bral1: its role in diffusion barrier formation and conduction velocity in the CNS. J Neurosci 2010; 30:3113-3123.
Brakebusch C, Seidenbecher CI, Asztely F, Rauch U, Matthies H, Meyer H, Krug M, Böckers TM, Zhou X, Kreutz MR, Montag D, Gundelfinger ED, Fässler R. Brevicandeficient mice display impaired hippocampal CA1 long-term potentiation but show no obvious deficits in learning and memory. Mol Cell Biol 2002; 22:7417-7427.
Chuzhanova N, Abeysinghe S, Krawczak M, Cooper D. Translocation and gross deletion breakpoints in human inherited disease and cancer II: Potential involvement of repetitive sequence elements in secondary structure formation between DNA ends. Hum Mut 2003; 22:245-251.
De Fusco M, Becchetti A, Patrignani A, Annesi G, Gambardella A, Quattrone A et al. The nicotinic receptor β2 subunit is mutant in nocturnal frontal lobe epilepsy. Nat Genet 2000; 26:275-276.
Drögemüller C, Becker D, Brunner A, Haase B, Kircher P, Seeliger F, Fehr M, Baumann U, Lindblad-Toh K, Leeb T. A missense mutation in the SERPINH1 gene in Dachshunds with osteogenesis imperfecta. PLoS Genet 2009; 5:e1000579.
Engel AG, Banker BQ. Ultrastructural changes in diseased muscle. In: Engel AG, Franzini-Armstrong CF (eds.). Myology 3rd ed. McGraw-Hill, New York, 2004, pp. 749-887.
Garosi LS, Platt SR, Shelton GD. Hypertonicity in Cavalier King Charles spaniels. J Vet Intern Med 2002; 16:330.
Gill JL, Capper D, Vanbellinghen JF, Chung SK, Higgins RJ, Rees MI, et al. Startle disease in Irish wolfhounds associated with a microdeletion in the glycine transporter GlyT2 gene. Neurobiol Dis 2011; 43:184-189.
Gleghorn L, Ramesar R, Beighton P, Wallis G. A mutation in the variable repeat region of the aggrecan gene (AGC1) causes a form of spondyloepiphyseal dysplasia associated with severe, premature osteoarthritis. Am J Hum Genet 2005; 77:484-490.
Harvey RJ, Topf M, Harvey K, Rees MI. The genetics of hyperekplexia: more than startle! Trends Genet 2008; 24:439-447.
Herrtage ME & Palmer AC. Episodic falling in the cavalier King Charles spaniel. Vet Rec 1983; 112:458-459.
Hirakawa S, Oobashi T, Su WD, Yoshioka H, Murakami T, Arata J, Ninomiya Y. The brain link protein-1 (BRAL1): cDNA cloning, genomic structure, and characterization as a novel link protein expressed in adult brain. Biochem Biophys Res Commun 2000; 276:982-989.
Karlsson EK, Baranowska I, Wade CM, Salmon Hillbertz NH, Zody MC, Anderson N et al. Efficient mapping of mendelian traits in dogs through genome-wide association. Nat Genet 2007; 39: 1321-1328.
Laing NG, Wilton SD, Akkari PA, Dorosz S, Boundy K, Kneebone C et al. A mutation in the alpha tropomyosin gene TPM3 associated with autosomal dominant nemaline myopathy NEM1. Nat Genet. 1995; 9:75-79.
Maeda N, Fukazawa N, Ishii M. Chondroitin sulfate proteoglycans in neural development and plasticity. Front Biosci 2010; 15:626-644.
Matthews E, Hanna MG. Muscle channelopathies: does the predicted channel gating pore offer new treatment insights for hypokalaemic periodic paralysis? J Physiol 2010; 588:1879-1886.
Miyamoto T, Inoue H, Sakamoto Y, Kudo E, Naito T, Mikawa T et al. Identification of a novel splice site mutation of the CSPG2 gene in a Japanese family with Wagner syndrome. Invest Ophthalmol Vis Sci 2005; 46:2726-2735.
Oohashi T, Hirakawa S, Bekku Y, Rauch U, Zimmermann DR, Su WD et al. Bral1, a brainspecific link protein, colocalizing with the versican V2 isoform at the nodes of Ranvier in developing and adult mouse central nervous systems. Mol Cell Neurosci 2002; 19:43-57.
Patterson EE, Minor KM, Tchernatynskaia AV, Taylor SM, Shelton GD, Ekenstedt KJ, Mickelson JR. A canine DNM1 mutation is highly associated with the syndrome of exercise-induced collapse. Nat Genet 2008;40:1235-1239.
Purcell S, Neale B, Todd-Brown K, Thomas L, Ferreira MA et al. PLINK: a tool set for whole-genome association and population-based linkage analyses. Am J Hum Genet 2007; 81:559-575.
Rusbridge C. Neurological diseases of the Cavalier King Charles spaniel. J Small Anim Pract 2005; 46:265-272.
Rees MI, Lewis TM, Kwok JB, Mortier GR, Govaert P, Snell RG et al. Hyperekplexia associated with compound heterozygote mutations in the β-subunit of the human inhibitory glycine receptor (GLRB). Hum Mol Genet 2002; 11:853-860.
Rees MI, Harvey K, Pearce BR, Chung SK, Duguid IC, Thomas P et al. Mutations in the gene encoding GlyT2 (SLC6A5) define a presynaptic component of human startle disease. Nat Genet 2006; 38:801-806.
Schouten JP, McElgunn CJ, Waaijer R, Zwijnenburg D, Diepvens F, Pals G. Relative quantification of 40 nucleic acid sequences by multiplex ligation-dependent probe amplification. Nucleic Acids Res 2002; 30:e57.
Shiang R, Ryan SG, Zhu YZ, Hahn AF, O'Connell P, Wasmuth JJ. Mutations in the α1 subunit of the inhibitory glycine receptor cause the dominant neurologic disorder, hyperekplexia. Nat Genet 1993; 5:351-358.
Stattin EL, Wiklund F, Lindblom K, Onnerfjord P, Jonsson BA, Tegner Yet al. A missense mutation in the aggrecan C-type lectin domain disrupts extracellular matrix interactions and causes dominant familial osteochondritis dissecans. Am J Hum Genet 2010; 86:126-137.
Thomas RH, Stephenson JBP, Harvey RJ, Rees MI. Hyperekplexia: Stiffness, startle and syncope. J Ped Neurol 2010; 8:11-14.
Tomlinson SE, Hanna MG, Kullmann DM, Tan SV, Burke D. Clinical neurophysiology of the episodic ataxias: insights into ion channel dysfunction in vivo. Clin Neurophysiol. 2009; 120:1768-1776.
Weber, Y.G., Lerche, H., 2009. Genetics of paroxysmal dyskinesias. Curr Neurol Neurosci Rep. 9, 206-211.
Wright JA, Brownlie SE, Smyth JB, Jones DG, Wotton P. Muscle hypertonicity in the Cavalier King Charles spaniel-myopathic features. Vet Rec 1986; 118:511-512.
Wright JA, Smyth JB, Brownlie SE, Robins M. A myopathy associated with muscle hypertonicity in the Cavalier King Charles spaniel. J Comp Pathol 1987; 97:559-565.

## Claims

1. A method for determination of the genetic disposition for episodic falling syndrome in a dog
**characterized in that** the presence or absence of a 15.7 kilo bases deletion in the brevican gene (*BCAN*) is detected in a DNA sample.

2. Method according to claim 1, **characterized in that** the deletion comprises at least one of the following elements of *BCAN*: regulatory sequences, exon 1, exon 2 and/or exon 3.

3. Method according to one of the claims 1 or 2,
**characterized in that** the deletion comprises at least exon 1, exon 2 and exon 3.

4. Method according to one of the claims 1 to 3,
**characterized in that** the deletion starts 1.56 kb downstream of *HAPLN2* and finishes 85 bp downstream of *BCAN* exon 3.

5. Method according to one of the claims 1 to 4,
**characterized in that** the deletion leads to a sequence according to Seq-ID No. 1 or Seq-ID No. 2.

6. Method according to one of the claims 1 to 5,
**characterized in that** the dog is a Cavalier King Charles spaniel.

## Patentansprüche

1. Verfahren zur Erkennung der genetischen Disposition für das Episodic-Falling-Syndrom in einem Hund,
**dadurch gekennzeichnet,**
**dass** das Vorhandensein oder Fehlen einer 15,7 Kilobasen-Deletion im Brevican-Gen (BCAN) in einer DNA-Probe nachgewiesen wird.

2. Verfahren nach Anspruch 1,
**dadurch gekennzeichnet,**
**dass** die Deletion zumindest eines der folgenden Elemente des BCAN-Gens umfasst: regulatorische Sequenzen, Exon 1, Exon 2 und/oder Exon 3.

3. Verfahren nach einem der Ansprüche 1 oder 2,
**dadurch gekennzeichnet,**
**dass** die Deletion zumindest Exon 1, Exon 2 und Exon 3 umfasst.

4. Verfahren nach einem der Ansprüche 1 bis 3,
**dadurch gekennzeichnet,**
**dass** die Deletion 1,56 Kilobasen (kb) strangabwärts (engl.: *downstream*) des HAPLN2-Gens beginnt und 85 Basenpaare (bp) strangabwärts des BCAN-Exons 3 endet.

5. Verfahren nach einem der Ansprüche 1 bis 4,
**dadurch gekennzeichnet,**
**dass** die Deletion zu einer Sequenz nach Seq-ID No. 1 oder Seq-ID No. 2 führt.

6. Verfahren nach einem der Ansprüche 1 bis 5,
**dadurch gekennzeichnet,**
**dass** es sich bei dem Hund um einen Cavalier King Charles Spaniel handelt.

## Revendications

1. Procédé destiné à la détermination de la disposition génétique pour le syndrome de chute épisodique (Episodic Falling Syndrome) dans un chien,
**caractérisé en ce que**
la présence ou absence d'une délétion de 15,7 kilobases dans le gène de la brevicane (BCAN) est détectée dans un échantillon d'ADN.

2. Procédé selon la revendication 1,
**caractérisé en ce que**
la délétion comprend au moins un des éléments suivants du gène BCAN : des séquences régulatrices, l'exon 1, l'exon 2 et/ou l'exon 3.

3. Procédé selon l'une quelconque des revendications 1 ou 2,
**caractérisé en ce que**
la délétion comprend au moins l'exon 1, l'exon 2 et l'exon 3.

4. Procédé selon l'une quelconque des revendications 1 à 3,
**caractérisé en ce que**
la délétion commence à 1,56 kilobases (kb) en aval (angl. *downstream*) du HAPLN2 et finit à 58 paires de bases (pb) en aval de l'exon 3 du BCAN.

5. Procédé selon l'une quelconque des revendications 1 à 4,
**caractérisé en ce que**
la délétion mène à une séquence selon la Seq-ID No. 1 ou la Seq-ID No. 2.

6. Procédé selon l'une quelconque des revendications 1 à 5,
**caractérisé en ce que**
le chien est un Cavalier King Charles Spaniel.


### SEQUENCE LISTING

<110> Laboklin GmbH & Co. KG
<120> A canine BCAN microdeletion associated with Episodic Falling Syndrome
<130> ADTPT12017EP
<150> EPEP11165620.3
   <151> 2011-05-11
<160> 64
<170> BiSSAP 1.0
<210> 1
   <211> 34
   <212> DNA
   <213> Canis lupus familiaris
<220>
   <221> source
   <222> 1..34
   <223> /mol_type="DNA" /note="Fig. 3d upper sequence" /organism="Canis lupus familiaris"
<400> 1
   aagtacaacc gccaggcctt agctagaaaa ggag 34
<210> 2
   <211> 34
   <212> DNA
   <213> Canis lupus familiaris
<220>
   <221> source
   <222> 1..34
   <223> /mol_type="DNA" /note="Fig. 3d lower sequence" /organism="Canis lupus familiaris"
<400> 2
   ctccttttct agctaaggcc tggcggttgt actt 34
<210> 3
   <211> 25
   <212> DNA
   <213> Canis lupus familiaris
<220>
   <221> source
   <222> 1..25
   <223> /mol_type="DNA" /note="Fig. 3e upper left sequence" /organism="Canis lupus familiaris"
<400> 3
   ctcagacctg gaagtacaac cgcca 25
<210> 4
   <211> 25
   <212> DNA
   <213> Canis lupus familiaris
<220>
   <221> source
   <222> 1..25
   <223> /mol_type="DNA" /note="Fig. 3e upper right sequence" /organism="Canis lupus familiaris"
<400> 4
   gtatgtacag gatcagcccc agttc 25
<210> 5
   <211> 25
   <212> DNA
   <213> Canis lupus familiaris
<220>
   <221> source
   <222> 1..25
   <223> /mol_type="DNA" /note="Fig. 3e lower left sequence" /organism="Canis lupus familiaris"
<400> 5
   caaatgctct ggaaggcacc cagtg 25
<210> 6
   <211> 25
   <212> DNA
   <213> Canis lupus familiaris
<220>
   <221> source
   <222> 1..25
   <223> /mol_type="DNA" /note="Fig. 3e lower right sequence" lorganism="Canis lupus familiaris"
<400> 6
   agctagaaaa ggagtgggta gagac 25
<210> 7
   <211> 350
   <212> DNA
   <213> Canis lupus familiaris
<220>
   <221> source
   <222> 1..350
   <223> /mol_type="DNA" /note="Fig. 4 first sequence" /organism="Canis lupus familiaris"
<220>
   <221> misc_feature
   <222> 181..350
   <223> /note="begin of deletion"
<400> 7
   cagaagggaa aagtaagaag agctctgaaa ggtcttacac ctgcaatgaa tagctgcagc 60
   gcagagagac acctgtctct cccctcgaca gctgtttctc tagaacgaat cccatggcct 120
   tgcttaacca aaagggggca gaaaagagca acctactcag acctggaagt acaaccgcca 180
   gtatgtacag gatcagcccc agttccacca caggcatcag ggcgggcaga ctggtgggca 240
   cccactcctc cccggctctt ctgggttttc tgagttacag tctgtacgga gtcagagccc 300
   cagaaagcca agacgctgga cccagaagct tccctgaagg aagatggctt 350
<210> 8
   <211> 490
   <212> DNA
   <213> Canis lupus familiaris
<220>
   <221> source
   <222> 1..490
   <223> /mol_type="DNA" /note="Fig. 4 second sequence incl. exon 1" /organism="Canis lupus familiaris"
<220>
   <221> exon
   <222> 82..417
   <223> /note="exon 1"
<400> 8
   gcactcgctc ccgcccctcc agccctcccc cctactccaa gtccccggct tctctccaga      60
   accatgtcct acttcgcacg tccttgctct ctctgctgcc tctaggaacc gacgcagagg     120
   aggcagcggt agcgtgacct tcccgagccc cctgcgtccc tggacagggg gcgggggcgg     180
   ccccggggag gagcggggcg gggacgggcg ggggagtgga gaaaggggtt ttgtgcgagc     240
   ccggcggccc ggcgccctct tccgaacgtc ctgcgggccc ggccctccct gcgcccgcgc     300
   agtcctcgcc gcagtcggcc gcagccgcag gacggagcgt ggacccggag gagagcccgg     360
   aggaggctgc aaacttggcg gtgcagcacc ctcgcagccc cggccgcctg caggtaggtg     420
   gagcacgagc gcagccggac caccgggacc cgagaggcag ggagcccggg gcgccgtcgg     480
   ccgccggtgg                                                            490
<210> 9
   <211> 280
   <212> DNA
   <213> Canis lupus familiaris
<220>
   <221> source
   <222> 1..280
   <223> /mol_type="DNA" /note="Fig. 4 third sequence incl. exon 2" /organism="Canis lupus familiaris"
<220>
   <221> exon
   <222> 84..181
   <223> /note="exon 2"
<400> 9
   agtccacatg gatgtggtca ggagccgggg gaagtccatc ctaaagtcta accccagctt      60
   tcctcctcaa gtccctccac cagcctgagc atggcccccc tgttcctgcc cctgctggta     120
   gccttggccc tggcccgggg ccctgtggcc ttagctgatg ccctggaagg ggacagctca     180
   ggtaagcaac cgcactcagc atcactgtct ctgtctcctg catctcctct atggaagagg     240
   gtggccttgg aggacccggg gagagatagg atagagggca                           280
<210> 10
   <211> 1050
   <212> DNA
   <213> Canis lupus familiaris
<220>
   <221> source
   <222> 1..1050
   <223> /mol_type="DNA" /note="Fig. 4 fourth sequence incl. exons 3 and 4" /organism="Canis lupus familiaris"
<220>
   <221> exon
   <222> 113..487
   <223> /note-'exon 3"
<220>
   <221> exon
   <222> 782..956
   <223> /note="exon 4"
<400> 10
   tggggccctg ggagcagtgg tggctgggat cctggggcgg gcttggagac caggtgggac      60
   cctgtggatc aggtgccaag ttctagccac cggcctgccc gcccacccac agaggaccgg     120
   gccttccacg tgcgcatcgc gggtgacgca ccactgcagg gcgtgctggg cggcgccctc     180
   accatccctt gccacgttca ctacctacgg ccgctgccgg gccaccgggc cgtgctgggc     240
   tccccgcggg tcaagtggac cttcctgtcc gggggccgtg aggccgaagt attggtggct     300
   cgggggctgc gcgtcaaggt gagcgaggcc taccgtttcc gtgtggcact gcctgcctac     360
   ccggcatcac tcaccgacgt gtccctggtg ctgagtgagc ttcggcccaa cgactccggc     420
   atctaccgct gcgaggtcca gcacggcata gatgacagca gcgatgccgt ggaggtcaag     480
   gtcaaaggtg aggggcagga cccaggaagg ttcccccagg gtgggagccc acagtgtgag     540
   gggggagcaa atgctctgga aggcacccag tgagctagaa aaggagtggg tagagacaga     600
   cctttgttac ctcctttctc tcttggggtg gacagactcc tgagcacagc ctggggcagg     660
   cccctcagca gtagagagag cacaatgtga actttaccct cgtgtacagg ggagtgccaa     720
   ggagggtgag aggaggtcag ggtggggtga tcatgcctca gggctcctct ctgcccctca     780
   ggggtcgtct ttctctaccg ggaaggctct gcccgctatg ctttctcttt cgctggggcc     840
   caggaggcct gtgcccgcat tggagcccgc attgccaccc cggagcagct ctatgccgcc     900
   tacctcgggg gctatgaaca gtgtgatgct ggctggctat ctgaccagac cgtgaggtga     960
   gcagggttgg gacgggggat cctgtggacc aggagcttct agttgtgtct ggaagtggca    1020
   gtgggccctg gttctgccag gggctgctgg                                    1050
<210> 11
   <211> 170
   <212> DNA
   <213> Canis lupus familiaris
<220>
   <221> source
   <222> 1..170
   <223> /mol_type="DNA" /note="Fig. 4 1st sequence deletion" /organism="Canis lupus familiaris"
<400> 11
   gtatgtacag gatcagcccc agttccacca caggcatcag ggcgggcaga ctggtgggca 60
   cccactcctc cccggctctt ctgggttttc tgagttacag tctgtacgga gtcagagccc 120
   cagaaagcca agacgctgga cccagaagct tccctgaagg aagatggctt 170
<210> 12
   <211> 572
   - <212> DNA
   <213> Canis lupus familiaris
<220>
   <221> source
   <222> 1..572
   <223> /mol_type="DNA" /note="Fig. 4 4th sequence deletion" /organism="Canis lupus familiaris"
<400> 12
   tggggccctg ggagcagtgg tggctgggat cctggggcgg gcttggagac caggtgggac 60
   cctgtggatc aggtgccaag ttctagccac cggcctgccc gcccacccac agaggaccgg 120
   gccttccacg tgcgcatcgc gggtgacgca ccactgcagg gcgtgctggg cggcgccctc 180
   accatccctt gccacgttca ctacctacgg ccgctgccgg gccaccgggc cgtgctgggc 240
   tccccgcggg tcaagtggac cttcctgtcc gggggccgtg aggccgaagt attggtggct 300
   cgggggctgc gcgtcaaggt gagcgaggcc taccgtttcc gtgtggcact gcctgcctac 360
   ccggcatcac tcaccgacgt gtccctggtg ctgagtgagc ttcggcccaa cgactccggc 420
   atctaccgct gcgaggtcca gcacggcata gatgacagca gcgatgccgt ggaggtcaag 480
   gtcaaaggtg aggggcagga cccaggaagg ttcccccagg gtgggagccc acagtgtgag 540
   gggggagcaa atgctctgga aggcacccag tg 572
<210> 13
   <211> 15722
   <212> DNA
   <213> Canis lupus familiaris
<220>
   <221> source
   <222> 1..15722
   <223> /mol_type="DNA" lorganism="Canis lupus familiaris"
<220>
   <221> exon
   <222> 10684..11019
   <223> /note="Exon 1"
<220>
   <221> exon
   <222> 14604..14701
   <223> /note="Exon 2"
<220>
   <221> exon
   <222> 15263..15637
   <223> /note="Exon 3"
<400> 13
   gtatgtacag gatcagcccc agttccacca caggcatcag ggcgggcaga ctggtgggca      60
   cccactcctc cccggctctt ctgggttttc tgagttacag tctgtacgga gtcagagccc     120
   cagaaagcca agacgctgga cccagaagct tccctgaagg aagatggctt cccttctgtg     180
   tgatgccctc gactttgtca agaaactgca taaaaaatgg tcacaggact ttacatttgc     240
   tgcaaatctt tctctctctc tctctctttt taaagatttt atttacttat tcacgagaga     300
   cacacagaga ggcagagaca taggcagagg gataagcagg ctccctgcgg gtagcccgat     360
   gcaggactcg atcccaggac cccaggatca tgacctgagc caaaggcaga cactgaacca     420
   ctgagccatc caggcatccc aaagctttat attttcaccc atgatagcac atgcccacta     480
   atttctacaa ctacgcccca cactactatt acaatgtttt tattacggag aacttcaaac     540
   atacgcaaga aaagagagaa aggatagtga accacttcat catccagctt gaccacttca     600
   aaattccacc tttctggttt catgaagtcc ctcacttgtg gtcgtttgtg gtggagtatg     660
   atttttttaa gattttattt atttattcat gagagacaca gagagaagca gagacacagg     720
   cggagggaga agcaggctcc atgcagggag cccaatgtgg gactcagtcc tgggtctcca     780
   ggatcacacc ctggcggaag gcggcactaa acccctgagc cacccgggct gcccatgctg     840
   gagtatttta aagcaaatct cagacagctt attattttct attaatcttc acatacatgc     900
   tatgcatttc taacatacgg tgactttaaa agataactaa ttctgttatc atattccccc     960
   aattgacgtt atctaatata tacttcatga tcagtttccc ttaaatgtct aaaaaatacc    1020
   attttatagt tgatttgccc aaaccagcat ccaagggcta tacattgcat ttgattaact    1080
   ttgtttattt tttaaaatat tttatttatt catttagaga gcatgagaga gagagagagt    1140
   aagcacacgc aagtgagaga aggggcagag tgagaatctc aagcagactc ctctctgagt    1200
   gtctggatct caggatctca tgacctgagc caaaatcaag agttggatgc taaaccaact    1260
   tagccactcc tgcgcctctt ggttaatttc ttaaaaatat ctttttaggg atccctgggt 1320
   ggcgcagcgg gttagcgcct gcctttggcc cagggcgcca tcctggagac ccgggatcaa 1380
   atcccacatc gggctctcgg tgcatggagc ctgcttctcc ctctgcctgt gtctctgcct 1440
   ctctctctct ctgtgactat cataaataaa taaaattaaa aaaaaatatc tttttagact 1500
   gtaatacttt ttcctttatg ttttctatgc catttttgtt tgtttaggaa actgggctgt 1560
   ttccctgtcg agtctcccat gttctagatc tggctgtgag tgtatccctg ttgtgtcatt 1620
   tcacatgttc ctctgtccct gtcattatat ttcactgttt gtttttctaa gattttattt 1680
   atttattcat aagagacaca cacacagaga ggcagagaca taggcaaagg gagaagcaga 1740
   ctccatgcag aaagcctgat gtgggactca atcctgggac tccaggatca caccctgacc 1800
   gaaaggcaga cactcaacca ctgagccacc caggagtccc atatatttca cagtttgatt 1860
   aaacatgcat taactttttt cccaggagta tttcataagt aagactgtat ttcctctgtt 1920
   gtcagatccg gaggtatgcg gtgtctggtt gttgatgcta cgactggtca gttaattctg 1980
   ctgctgtcag cctgatccac tcacaaagtt gcttgtacca gttatgcagg ctgctgcaac 2040
   acattaccac aaacgtggtg gtttatgagc cagaaatata ttcttctact gttctggagg 2100
   ccagaagtct aaactcagag tgttggcatt atggccctcc ctctgtgtag actctgagag 2160
   aattcttcct tgcttcttcc atcttcagtg gcaggtggca ttcctcagtg tctctggctg 2220
   cattgctcct atctctgtgt ctgtggtcat gttgcctcct ccttttcatg caatacgacc 2280
   acagacagag ataggagcaa tgcagacaca ttaaactgaa ttttgggaat aaattcatgt 2340
   aatgaattac atccacagag accctttttc caaacaaggg tttcagggat taggatgcag 2400
   acacatcttt acccatcacc tttttatctg atggctactg gtgaccatgg tctagatgca 2460
   ttatttcatt aggagcacaa aatggagatg ttctaatttt gtctctcctc ttcctttatt 2520
   attatgaatt gtcccatatg gaaaaatatc cctcatcaat tactgtcact ctggcttaga 2580
   acccattcag gaaagataga gttaatgcct ggttctctcc catttgtctt attttttttc 2640
   ccatttgtct tagataggta ctgtggaatg agtgtttgca tcacccaaat ccatatatta 2700
   aagccctaat ccccagtgtg gtggttttgg acatggggcc tttggaaggt aatcccacga 2760
   tgacattagt gcccttataa gaagagatgt gggagagctt gctctctcct tgcagacttc 2820
   agtgagaagg caggcgtctg caggaagcca gccctcacca gacaccaggt ctcctggcct 2880
   ccagggatga gagaaatatg tgtttgttgt ttaaaccatc caggctgggg cacctgggtg 2940
   gctcagttgg ttgagcatct gcctttggct caggtcgaga tcccggggtc ctgggatcca 3000
   gtcccacatt ggacttcctg cgcggagcct gcttttccct ctgcctatgt ctctgcctct 3060
   ctgtgtcttt catgaataaa taaataaaat cttaaaataa ataaacaaaa aaacaaataa 3120
   ataaacaaac catccaggct atggtaactt gttacagcag cctgagctga ctaagaccat 3180
   ggggttccct gaaaccaacg ttgagtcaaa gatttcagta caagcagttt atctgggggg 3240
   tcaggtaaaa cgcaccagtg ggggactggg gaagcaaggc aggagggagg aggaagctgg 3300
   caaaggctgt gagacagagt ggccggagcg attggcccag tcctgccaag ggatccctgg 3360
   aaggcagtgt ggagcctgtc tcagagaatc ccacctgatg gacagacagc agggtcatat 3420
   gtgtcaactt cctgcctgaa agccgagggc tgcttctggg agcactgacc cgttagcatt 3480
   cctggctagc tctgagtgca cacatgctcc cacagtgaga aaaaagccac cagacagaat 3540
   cccagatgct accgtctgcc tatggagaca aatactggag ggtcgggtca cagctgtggt 3600
   ccaccctccc cactgctcag acccatgtgt ccccctgtcc accgtgtcct gtcactgact 3660
   ctccaaggct gtggctgggc accattttta attcttgaaa agagagtttt atgggcagcc 3720
   ccagggggcc cagtggttta gcatggcctt cagcccaggg ccttcagccc agggcgtgat 3780
   cctggagacc cgggatcgag tctcacttca ggctccctgc atggagcctg cttctccctc 3840
   tgcctgtgtc tctgcctctc tctctctctc tctctctgtg tctctcatgg ataaataaat 3900
   aaaatcttaa aaaagaaaga aagaaaacag agtcttatga caggagggtt agtgggacaa 3960
   gttccaatag ttgctgcagc gggtggctcc ggggctgtga ccattatcaa tattccacat 4020
   cttcctccat ggttcatctg ttgtccacag ggctttcaca gaaaaaccca gaccttcatc 4080
   cctgaaggcc tttacttccc atgcccttga caggccagca tggctgcacc tgccatcata 4140
   cttaccaccg gatatgggct cgtcaagagt aaatgttttg gggtgcctgg gtggctcagt 4200
   gattgagcgt ctgcctttgg cttgggttgt gatcctgggg tcctgggatt gggtgccctg 4260
   tttggctcgc cacagggagc cttcttctcc cactgcctgt atctctgcct ctctctgggt 4320
   gtctctcgtg aataaataca cttttaaaat cttaaaaaag ggcagcccca gtggcgcagc 4380
   ggtttggcgc cacctgcagc ctggggtgtg atcctggaga cccgggatca agtcccacat 4440
   caggcttcct gcatggagcc tgcttctccc tctgtgtctc tgcccctctc tctgtgtcta 4500
   tgaataaaca aataaaatct taacaataaa ataaaataaa ataaaatctt taaaaaaaga 4560
   acaaaagtct ttgccagtat aactcttttc tttgcgtgtt ggtgaattgg catgaggtac 4620
   ccaaaaggac ccagaggctg ctagtacctt aggtctggtg gtacccttat agtagcctct 4680
   ggcagacaca tctcccaccc taaggactag ggcttctaat cctacagagt ctacatttat 4740
   aggtacagga agcacgaatc ttcacatggg tcactgggga tgatggtggg aaattattgc 4800
   tctttccaac ccttggttct tgtatccatg acttctagct ctgtgggcta cagcaccata 4860
   taatagccat tggttcaaaa tgtagactgc attttgaagt ccagtactct tgatttcaga 4920
   ggatgttttc cccaaactgg tatttagatg tgctgtaacg aggtcatact aaagcactcc 4980
   cagggttggc aatttcaagg tcatgtaata catggtaggg acaatggatg cggtggtctt 5040
   gtggccattg tcgcatctcc tttactttaa agtgtatccc cttggttaga agtgatacta 5100
   tatggggtct cataagtata agccatctcc ttcatgacct tgaatattgg tactggccaa 5160
   ggcacttggg cagggaaggc aaattcatac ccagaatgtg catagtccca ggaaggaaac 5220
   attgccctcc catccagact gaaagagaga cactgtagtc aacatgcttc ccagtggctg 5280
   gttgatctcc ctgagggatg gcgccatgtg agggctcagc acctgtctgt gctgccgaca 5340
   cgtcaggtgg ccttagtgag agggaaccat gcagacttct acccactgcc atagccactc 5400
   cattcatggg ccctcacgca ggcactggca tgggggaagg acagagtctg gctggtatcc 5460
   cctggagtct cctgtctcct ggctgtccaa cgttcccctg cagtgaatgc tccttaatgg 5520
   tactgctgta aaaacaatgc ccgctcctgc aagtccatcc atggctctcc ctccacttcc 5580
   ttatctccac ccttcctgtc ctgccctttc ctggcctgac cagccagcca aaccatgtgc 5640
   caccgctcaa gagtccaata gacctctctc aagtcacctc tccccctgca ggcaagggtg 5700
   gccagcgtgc tgcctgaact ccctccccag cctccatccc gggtcagctc acatcagtgc 5760
   accagactga tccatccatg aagtaggcct ggacttcctc ctctgaagcc tgattggcct 5820
   gatcacagag gcactgagag agaggcatgg gtgccacaga cacagggcat ctgggagcct 5880
   ggcttcctgc tctctgagca gtctcaccca atcacacatt ccacctggga ttgccgttgt 5940
   gtccacccat atgtatgtgc aggctttgaa cccccaggtc ttaataagac agtgagtcca 6000
   ggaagggaga gaatccaaca aatcattgag caaacagtcc aatcacctgg aacaggtgcc 6060
   cagctggagc tcaaagctgc tgagagtgaa gggagagggg ggcggggtgg ggcttctagt 6120
   ctgcgggctc cccttctgtc actggccgaa gctgtttcca ggccatcaac tcctacatgc 6180
   gtctgactcg ctgtgttctt gggcccagtg tacctgccca ttcagaaaaa atcctcagga 6240
   agtcttcagc ctgtggcctg tctaggtggc cagtaagggg acataggcca gtaaggggac 6300
   actgacaggg tctgctacac cctcttaatc ttcacaactg tgagtccatg cccccatcag 6360
   tgtgcaaagg tgatcaggga ttttcttgtt tagtatcatg atgaattctt ggactttata 6420
   gatttgatgt gcttccatct cttatgggat ttattcattt aatgctgaca tgtcaccttg 6480
   ccagtgagag tctccacaat gtggctactg ggtccctttg acatgatctg acctgagacc 6540
   ccaaacttcc tttatttgca agtgtgatga gctgtcctgc ccctgactgg gacatcctga 6600
   agaggcctgg ttcctggaag tggaaagtac tatttagaga cggtcgtctg gaccccagga 6660
   gcactcagca ttaacggggt gggtgggggg tagtgtcact gcttctaagc ctttttcaca 6720
   gacatacaaa tcgtgagctc atgctgttgt cttcagttca cgtgaaagat tagcagttta 6780
   gcttaactct cctttcctca cactgaaact cagagttcca aacaccatta ctataattat 6840
   ttgtttgcat tatcccaata aatagtgcgt tttgaaatgc caagccaaca ttatgatttc 6900
   tctgcagttc ttttcattct tagggatgaa tagtcaaaat gctcagtttc ataatcactt 6960
   gaaagaaaat tcttccatgt ggctttgctt ccagctccaa gtacaatcac gggcccttgt 7020
   ctcactttgt ttcagatttt taggaattgc atccctgttc tgcttcttaa tttagcctaa 7080
   aattaacttc attattctga acttaattct gtttcaatta tataatatat ttatgtagtt 7140
   ccaatgtcaa agccagaaag caagctgctg ccattttaca ggtgaagaaa caggcctgca 7200
   gcctgctctt cagaaaacct gagaggtctc tggccctggg ctgtagggaa gggggctgcg 7260
   aggagcctgc agatgggacg gctttctcca gaaggagatc tggatgttct cagggccgag 7320
   ccctccagtt ccttcagctg ttctttgggg aacagtctgg gatgcccttt gagcatagga 7380
   ctttgctccc tggccgcaaa gttctgtcca ctcaggggct cccctagatg tggtgccgtt 7440
   cagcctgcct cctcgccacc ccccctcccc tacccaccct gaggccttca gctgcaggag 7500
   ccctccctca tctgggccag ggtcccgcgc ttcctggccc ctttcagaag ttctcaaagc 7560
   caccattctg ccagcacagc tgtggcagcg cttggagctg tgcgcatgca gccccactcc 7620
   cacgtccccc tgcagaggac ccaggtgccc acatgacgtg cagggccttg agaggatctg 7680
   agctgctggg ccccaccccc acgtctgggg aagtggaaac aggtgctcaa gggaccagaa 7740
   ggatgggctc tcccagtggc cactgtggct gggagtccag tgaccagtgg ctaacactgg 7800
   aggcgggggc gccagggacg ccaggacctt ggggagggtc tgtttcctca acttgggtca 7860
   tggcctcagc gttggccttg gtgggggggc cgtgggggtg agggagaggg aggagactga 7920
   ttattctcct ccacagagac tggaaagctc cccaggaccc aggaggctgg gaggccagcc 7980
   agctgggctg caaccccctc ccccccacct ccaccatttc tgtcccctga gagacagctg 8040
   gggccctggc aagaaccctg gcctgggcgt ccatcaggag acttgctttt ggtcatggtc 8100
   agcctgtgac atgctgtgtg accccagata actttccacc tctgggtctg tttccttgtc 8160
   cagaagatat ttcagtttcc ctgagacatt ctccttctgt tattagggca ggaagttcta 8220
   cgtgaaatca gacttgaatt cttcctgctt ccattggtcc tgtttgcttt cagccagtct 8280
   ccgagggtcc ctttctgaga cctcagggag gtcttggttc ttcttgcctc ttcttaggaa 8340
   gggatagaaa tgggtgatgg tgactgtcac atggccaggg gacaagatgc aggtaaaaat 8400
   agtacagaag gtccctgccc agcaagtgcc agctagactg cagtatgagt cttcagagct 8460
   gtaagtgttg gaagagtctc gggggcgagg gggcactcat tgcctcccct ggaaattcct 8520
   cagggcggac tcctctctca gataacaaac gctccgtctc acccacagga gactggttca 8580
   ctggcctcct ggccaggaga tctgtgcaca ctgggcatgt gtgtatgtgc gtgcatgact 8640
   agcaaagaga aagaactaga ttttgctgag ctcgtccatg ggccaggtgc agtacacatt 8700
   acccagtgcc attgatactc ttcagcccgt tgttcagaag aggaaagagt ttcagagaaa 8760
   ggaagccctg cacaaggttt ggccgctgca gatggcaaac cagggctttg acctcagggc 8820
   cggagcctct ggagtcctgg ctctcaccag ccttcacagc agcagctccc tgttcccgca 8880
   ccacgtctgg tttcccacca gggggactgc ctcttggaac agactgtctg gtaccagccc 8940
   cagtccagac ccagctgctt ctggcctcga tcaaggtcag actctttcag gatgccacgt 9000
   gtgcccacac ttctagtcat gaggggaggt gacaaggaag gtcactttct cttctttccc 9060
   catcacagcc aggtttcccc acagcaagaa ctgtggttac ctgggagcca ccactgagat 9120
   taaatttcag ccacatggtt gcctggctct gcctggctgg gcttaagacc cgggtggagc 9180
   tgatcccagc cctggtggag gcaggctggc tgcatggacc tatgctgaga ctgttggggt 9240
   gggggggatg tctctttcag ttgggttgca aggtccgagg ggagcaaagg gaggtcctag 9300
   ggctgccagg tgctgcttag atctgcccca aggccagatc cctctggcct ccatcagagg 9360
   acttggtgct gaaatagtgc ctggtccata gtgggcattc agttagtatt tatttaatga 9420
   aatgaataaa cagaggtctc tccttcccca gactctgcca ggcaccttgt tctgtcctct 9480
   ttaggctcac ctccccgcct gggtcatttc ttttactaaa agaccctacc acccaccacc 9540
   cccaggactt cctgtcctgt ctctacagta tatgggtgtc caaggattgg ccagagctct 9600
   accttaggtc cttgaggatt tagggaggag tttattgtga gcttctgaaa agcgctttgg 9660
   aagccagtcc acagcatttg aagtagaatc cagacagtgg taagactggg acagttaggg 9720
   catcagcaga gggcagtcca gagtcccata caggagggag ggacttgggt gccgtctaaa 9780
   tggaaggaca gggacctgtc ctgaggttga tttgcactgc aggcaccccg catgacttgg 9840
   gatattttcg gggtgtgctg gagattatgg ggatcaaatt ccccaacaag ctatccttgg 9900
   tgccaccaca gttatccggg gagagaattc aagcatctag ctgtcctgcc cacagtcagt 9960
   gccaggggct ctgcttcaag tcccaggagg gacaggggga gtgagaagag ggaggggcag 10020
   agagagcctg ggaaagcaga gggaagggga ggctgtggag ctgaagaggg ggggtgtcat 10080
   tggtattctg aggagtcccc cagtgctagg cagcactcaa ggcccacccc ctggcaccag 10140
   gctgcacatt catcagcaac agccctggcc agtgggccct ctgggctccc agcaacgcct 10200
   gtgttttgca cattggtttt tgtgaccagt gtgtccattc acagtgccgc tcctccccca 10260
   cacggcatcc agagagtccc tgacttgctt ttcttaaccc ctgaatccgc tgtctaacct 10320
   ccatctgtcc agctgcaaag tcagctcctc ctctgtgcag ggagcaggag caccaccaga 10380
   ggcaaaaccc aatcagggct tccaacaggg atgtggttgt ggtcctttgc ccctcatcct 10440
   gcctccaaaa aagagcttcc ctcccccact ctcatgcttc tggtatcctg tccctattgt 10500
   ccaattccca ggccctcctc ctcgcccctg gccccacggg aagtccctct ggctgtctaa 10560
   cttccatccg tccagttgca gcagcatcct ccagctccgc gagcactcgc tcccgcccct 10620
   ccagccctcc cccctactcc aagtccccgg cttctctcca gaaccatgtc ctacttcgca 10680
   cgtccttgct ctctctgctg cctctaggaa ccgacgcaga ggaggcagcg gtagcgtgac 10740
   cttcccgagc cccctgcgtc cctggacagg gggcgggggc ggccccgggg aggagcgggg 10800
   cggggacggg cgggggagtg gagaaagggg ttttgtgcga gcccggcggc ccggcgccct 10860
   cttccgaacg tcctgcgggc ccggccctcc ctgcgcccgc gcagtcctcg ccgcagtcgg 10920
   ccgcagccgc aggacggagc gtggacccgg aggagagccc ggaggaggct gcaaacttgg 10980
   cggtgcagca ccctcgcagc cccggccgcc tgcaggtagg tgagcacgag cgcagccgga 11040
   ccaccgggac ccgagaggca gggagcccgg ggcgccgtcg gccgccggtg gtccgcgccc 11100
   ccccctcccg cccccgcccc gcagctctgg cctcccgggg ctccggcgca ggccacctgc 11160
   tgaccctgac agcccccagg cgcccctccg ctgtgtgcgg cccctcccgc gcccccgagt 11220
   ccacggatga taggaacgcc ccccgtcact gcggcctcct tgctgcgccc tcgggcgcca 11280
   gcccccggct caggcttccg accttgtgcg gggcaggcgg gcggcgggag gagcaggaag 11340
   cagcccctca ttggaggccc cttcggggag ccccgggaca gccgacagcg gagcgggtgg 11400
   tcggggcgga ctggcgctgg agtcccaact ctgccactta cttgctgtgt gaccttgggc 11460
   aagttagttg acctccctga acctcgggtt cctcttcagt gtgatgcata gaccatattt 11520
   ttctcaaagt taaggttatc catgcgaagc ctggataaga ggcctgactc aggtggatgg 11580
   tcagtcacca gtagccagag ctccagttac cctcctgctt tcctctctag cactgccagg 11640
   gggctggaga cccccccccc aggtcctttc ccctcaccct cctatgggaa tagctctgcc 11700
   cacacgcttc caacaggacc aagagatttg ccatcagttt tgttagcacc gttaatatct 11760
   gcatgacttc tattccagat aagagcagta agatcaaggg acactgttac ccatggacca 11820
   cccaccccca ctcaccctgg ccccacagca ttaccagcac ctgtgttttc atggctaact 11880
   tggcatcatc cacctttcag attctgaact aggaggagct gcagtagctc agctgtgatg 11940
   tcgctaatag tattactagt tgtgcgtcca ataaatagga ctatttatgc aatgtcttta 12000
   gctggtccca caagctctgc taatgcctgg ctgctgtcgt gtaaggtcag gttgctgctt 12060
   gcacggccac taaggggacc ataagagctt ccctcctgct gaccctgcta gctacaggag 12120
   cctggttcct atggggcaga ctctgcacag ccagcaggga aggggggggc atttagcatg 12180
   gagatggcag gtattggatg tgtgtacgca tatgcatgga cacacacaca cacacacaca 12240
   cacacacaca cacacacaaa ccctggtcct gatcttaatc ctctgtggat tgggtctacg 12300
   aagcctgtga gaaagatggg ctccgtgatc agacaggaat aacatatcaa gagagaagtt 12360
   tcctgaacag cttgggaagc tcaaggcaag ggcagaaatc ctggagctgg agcaggaaag 12420
   gatgagctgg agagagggat ggtgtggatc agggaaggga caggaagcct gtcgatgccg 12480
   aaactctcca ggttagggat ggagagggta tcctgctctg gaggacagca gaggggtgaa 12540
   ggccaagtct gttttccctt tccccacccc aggtctgaca ctggtggcag aggaggggat 12600
   gcagtagtgt tctcaggaca cttggctgta ccaggatggg gctgaggccc aagcaatggg 12660
   gtgggggcaa tgcagatgtc tctgatcatg agaaccatgt aatccaggtg ccctcttggc 12720
   ccctctggtt agtgactccc cttcctggaa gtgggatggc tcccatattt cagggtacca 12780
   ggtcttcatc agcctcagac tttctccctt cattccctca ccttcaaaac agaggtaaaa 12840
   gggcaggcca gggtgctggt cccagagtgg ctccaaatat atgggtttct gggccgtctg 12900
   tcagagagct gggacccagg tctatgagca gctccagctc cagcctgaag agaggggagg 12960
   gagggcgcca gaaaggggcc tggcccagga cctggcacag ctcagatgca aggcaaacag 13020
   tctgcccagt acccagctgg caccggcttc ttaaaccaaa tggtccctcc ttctacccca 13080
   gaaggcagtc ctcccaagtg cccctatacc cagagctggt ctcagcaccc tctgatctgg 13140
   ccccaacaag agacaccctg gacatctggc tcctgtcact ccttctccaa gtctaaccct 13200
   gaggaaatga agcccagcca tagcgggagg gatggaggtg ttgtaggtgg ggaggcattc 13260
   caaggaggat ctcagatccc tgggaggatt tgggagaaga gaggtttccc agcagttcag 13320
   gctcagaaag gggaacacat gcatgaaggc agagcctaag agggcaaaga agatggaggt 13380
   tgaaccatgg gcctggagtg ggaatgagcc tctaggaaat cctgcctcta gactaacctg 13440
   catccctcct gctgcgccat tggcctatgc catggcagac ctgggtctct gtgcttttct 13500
   actagccctt acagatgttg ttggtcacta gctctgaagc cttccctcct gcaggcacat 13560
   tggcctggta cctgacttgg ctttgctggg aaccagagtc agccctatcc ttctctgggt 13620
   cccatttctc agtgtcaccc cccaacaccc ccatctctcc attatgaaag tgtaagtgct 13680
   ttgagatctt cagaggacta ttctgggttc tcagggtgtc tgagagccaa gggcaagagg 13740
   gagatgaccc ctcccattta gtctccccct cccttagccc acccccccag cctagggctg 13800
   tggtggggga agggagaggc tggatggagg ccagactggc agagacaggg gctttgtccc 13860
   agctggtttc tgagccagga gatccggtgg gaaagcccgt gccaggcaga aaggaaggct 13920
   gggaagaagt gagaaggcag ccaagggcgg aggggagtgg aggatgctaa gggtaaagag 13980
   aagagtttcc ttataagtac tggtggggta gggggaggtc aaggaggggt atagctagag 14040
   agaaggtgtg catcacccag gttcagtcta ggaggccctc ctggaagagg ctgggtgggt 14100
   atggtactca gggaggaagg ggcgggaagg aaaagttggc agggtcctgg ggctctgggt 14160
   tcaaggaagg agtctggcac tgtgaagacc agagggaatg aagtatgata gcacctacca 14220
   ctcatgctat aagcatttat tgagctccta cggtgtgcct ggttctgtgc atctgtgcag 14280
   cgcttatagg ataaaaggcg ctctagaacc cggagcctca tcaccaccca ggtgctgtcc 14340
   tacaagtcag gaaacaaagg cctacagagg ttaatgaatt agccttaagt cacacaggaa 14400
   gttgactaga acccaggtct tggttccaag gaagagtcct ctggtgggga gacagaatca 14460
   aaaacaaggg agttaacggt gaggttggga cagtgggctt cttggaccac catatgtgtg 14520
   agtccacatg gatgtggtca ggagccgggg gaagtccatc ctaaagtcta accccagctt 14580
   tcctcctcaa gtccctccac cagcctgagc atggcccccc tgttcctgcc cctgctggta 14640
   gccttggccc tggcccgggg ccctgtggcc ttagctgatg ccctggaagg ggacagctca 14700
   ggtaagcaac cgcactcagc atcactgtct ctgtctcctg catctcctct atggaagagg 14760
   gtggccttgg aggacccggg gagagatagg atagagggca ctgtggggaa tgatgtggtc 14820
   tgggatgctt ggaaataagg gataagctgc tggctctggg tagggcaggc aaaattagtg 14880
   gtaggactgc caacccctag ggcatgggac caatgaaggg ggctggaaga cagaacaggg 14940
   atagagtggg gagaaatccc aaaggtgagt ctgagggaaa ctgaggggaa actttaaggc 15000
   cagggctggg gccagcttag atctggagtg ggccccaggg agtcctggga tggtacctac 15060
   caggccactg tccacaggaa cagactggaa gatttgggga cagatctgga gagggagtgg 15120
   gatgggcctg gggtcctaga ggcaagcatg tggggccctg ggagcagtgg tggctgggat 15180
   cctggggcgg gcttggagac caggtgggac cctgtggatc aggtgccaag ttctagccac 15240
   cggcctgccc gcccacccac agaggaccgg gccttccacg tgcgcatcgc gggtgacgca 15300
   ccactgcagg gcgtgctggg cggcgccctc accatccctt gccacgttca ctacctacgg 15360
   ccgctgccgg gccaccgggc cgtgctgggc tccccgcggg tcaagtggac cttcctgtcc 15420
   gggggccgtg aggccgaagt attggtggct cgggggctgc gcgtcaaggt gagcgaggcc 15480
   taccgtttcc gtgtggcact gcctgcctac ccggcatcac tcaccgacgt gtccctggtg 15540
   ctgagtgagc ttcggcccaa cgactccggc atctaccgct gcgaggtcca gcacggcata 15600
   gatgacagca gcgatgccgt ggaggtcaag gtcaaaggtg aggggcagga cccaggaagg 15660
   ttcccccagg gtgggagccc acagtgtgag gggggagcaa atgctctgga aggcacccag 15720
   tg 15722
<210> 14
   <211> 336
   <212> DNA
   <213> Canis lupus familiaris
<220>
   <221> source
   <222> 1..336
   <223> /mol_type="DNA" /organism="Canis lupus familiaris"
<400> 14
   ccttgctctc tctgctgcct ctaggaaccg acgcagagga ggcagcggta gcgtgacctt 60
   cccgagcccc ctgcgtccct ggacaggggg cgggggcggc cccggggagg agcggggcgg 120
   ggacgggcgg gggagtggag aaaggggttt tgtgcgagcc cggcggcccg gcgccctctt 180
   ccgaacgtcc tgcgggcccg gccctccctg cgcccgcgca gtcctcgccg cagtcggccg 240
   cagccgcagg acggagcgtg gacccggagg agagcccgga ggaggctgca aacttggcgg 300
   tgcagcaccc tcgcagcccc ggccgcctgc aggtag 336
<210> 15
   <211> 98
   <212> DNA
   <213> Canis lupus familiaris
<220>
   <221> source
   <222> 1..98
   <223> /mol_type="DNA" /organism="Canis lupus familiaris"
<400> 15
   cctgagcatg gcccccctgt tcctgcccct gctggtagcc ttggccctgg cccggggccc      60
   tgtggcctta gctgatgccc tggaagggga cagctcag                              98
<210> 16
   <211> 375
   <212> DNA
   <213> Canis lupus familiaris
<220>
   <221> source
   <222> 1..375
   <223> /mol_type="DNA" /organism="Canis lupus familiaris"
<400> 16
   aggaccgggc cttccacgtg cgcatcgcgg gtgacgcacc actgcagggc gtgctgggcg      60
   gcgccctcac catcccttgc cacgttcact acctacggcc gctgccgggc caccgggccg     120
   tgctgggctc cccgcgggtc aagtggacct tcctgtccgg gggccgtgag gccgaagtat      180
   tggtggctcg ggggctgcgc gtcaaggtga gcgaggccta ccgtttccgt gtggcactgc      240
   ctgcctaccc ggcatcactc accgacgtgt ccctggtgct gagtgagctt cggcccaacg     300
   actccggcat ctaccgctgc gaggtccagc acggcataga tgacagcagc gatgccgtgg     360
   aggtcaaggt caaag                                                      375
<210> 17
   <211> 175
   <212> DNA
   <213> Canis lupus familiaris
<220>
   <221> source
   <222> 1..175
   <223> /mol_type="DNA" /organism="Canis lupus familiaris"
<400> 17
   gggtcgtctt tctctaccgg gaaggctctg cccgctatgc tttctctttc gctggggccc      60
   aggaggcctg tgcccgcatt ggagcccgca ttgccacccc ggagcagctc tatgccgcct     120
   acctcggggg ctatgaacag tgtgatgctg gctggctatc tgaccagacc gtgag           175
<210> 18
   <211> 25
   <212> DNA
   <213> Artificial Sequence
<220>
   <221> source
   <222> 1..25
   <223> /mol_type="DNA" /note="EFS1 multiplex PCR primer" /organism="Artificial Sequence"
<400> 18
   aaggtcttac acctgcaatg aatag 25
<210> 19
   <211> 25
   <212> DNA
   <213> Artificial Sequence
<220>
   <221> source
   <222> 1..25
   <223> /mol_type="DNA" /note="EFS2 multiplex PCR primer" /organism="Artificial Sequence"
<400> 19
   agcaaatgta aagtcctgtg accat 25
<210> 20
   <211> 25
   <212> DNA
   <213> Artificial Sequence
<220>
   <221> source
   <222> 1..25
   <223> /mol_type="DNA" /note="EFS3 multiplex PCR primer" /organism="Artificial Sequence"
<400> 20
   agttcacatt gtgctctctc tactg 25
<210> 21
   <211> 24
   <212> DNA
   <213> Artificial Sequence
<220>
   <221> source
   <222> 1..24
   <223> /mol_type="DNA" /note="BCAN exon 1 forward primer" /organism="Artificial Sequence"
<400> 21
   cttctctcca gaaccatgtc ctac 24
<210> 22
   <211> 24
   <212> DNA
   <213> Artificial Sequence
<220>
   <221> source
   <222> 1..24
   <223> /mol_type="DNA" /note="BCAN exon 1 reverse primer" /organism="Artificial Sequence"
<400> 22
   acacagcaag taagtggcag agtt 24
<210> 23
   <211> 25
   <212> DNA
   <213> Artificial Sequence
<220>
   <221> source
   <222> 1..25
   <223> /mol_type="DNA" /note="BCAN exon 2 forward primer" /organism="Artificial Sequence"
<400> 23
   gagttaacgg tgaggttggg acagt 25
<210> 24
   <211> 25
   <212> DNA
   <213> Artificial Sequence
<220>
   <221> source
   <222> 1..25
   <223> /mol_type="DNA" /note="BCAN exon 2 reverse primer" /organism="Artificial Sequence"
<400> 24
   cccacagtgc cctctatcct atctc 25
<210> 25
   <211> 25
   <212> DNA
   <213> Artificial Sequence
<220>
   <221> source
   <222> 1..25
   <223> /mol_type="DNA" /note="BCAN exon 3 forward primer" /organism="Artificial Sequence"
<400> 25
   aagatttggg gacagatctg gagag 25
<210> 26
   <211> 25
   <212> DNA
   <213> Artificial Sequence
<220>
   <221> source
   <222> 1..25
   <223> /mol_type="DNA" /note="BCAN exon 3 reverse primer" /organism="Artificial Sequence"
<400> 26
   ccccaagaga gaaaggaggt aacaa 25
<210> 27
   <211> 25
   <212> DNA
   <213> Artificial Sequence
<220>
   <221> source
   <222> 1..25
   <223> /mol_type="DNA" /note="BCAN exon 4 forward primer" /organism="Artificial Sequence"
<400> 27
   ctttctctct tggggtggac agact 25
<210> 28
   <211> 22
   <212> DNA
   <213> Artificial Sequence
<220>
   <221> source
   <222> 1..22
   <223> /mol_type="DNA" /note="BCAN exon 4 reverse primer" /organism="Artificial Sequence"
<400> 28
   ggtcctggat ggttgacctg ag 22
<210> 29
   <211> 25
   <212> DNA
   <213> Artificial Sequence
<220>
   <221> source
   <222> 1..25
   <223> /mol_type="DNA" /note="BCAN exon 5 forward primer" /organism="Artificial Sequence"
<400> 29
   gggtgtctct gcagaagaaa acaat 25
<210> 30
   <211> 21
   <212> DNA
   <213> Artificial Sequence
<220>
   <221> source
   <222> 1..21
   <223> /mol_type="DNA" /note="BCAN exon 5 reverse primer" /organism="Artificial Sequence"
<400> 30
   gaagacctct ggacagcacc g 21
<210> 31
   <211> 20
   <212> DNA
   <213> Artificial Sequence
<220>
   <221> source
   <222> 1..20
   <223> /mol_type="DNA" /note="BCAN exon 6 forward primer" /organism="Artificial Sequence"
<400> 31
   ggcccagaga agccagccta 20
<210> 32
   <211> 25
   <212> DNA
   <213> Artificial Sequence
<220>
   <221> source
   <222> 1..25
   <223> /mol_type="DNA" /note="BCAN exon 6 reverse primer" /organism="Artificial Sequence"
<400> 32
   gggaaacttc agagctcaag tctgt 25
<210> 33
   <211> 25
   <212> DNA
   <213> Artificial Sequence
<220>
   <221> source
   <222> 1..25
   <223> /mol_type="DNA" /note="BCAN exon 7 forward primer" /organism="Artificial Sequence"
<400> 33
   ccacagggaa gatgagtgag aattg 25
<210> 34
   <211> 22
   <212> DNA
   <213> Artificial Sequence
<220>
   <221> source
   <222> 1..22
   <223> /mol_type="DNA" /note="BCAN exon 7 reverse primer" /organism="Artificial Sequence"
<400> 34
   ccagcatcac tctggacacc tt 22
<210> 35
   <211> 20
   <212> DNA
   <213> Artificial Sequence
<220>
   <221> source
   <222> 1..20
   <223> /mol_type="DNA" /note="BCAN exon 8 forward primer" /organism="Artificial Sequence"
<400> 35
   ctcaccctcc acagcccctt 20
<210> 36
   <211> 25
   <212> DNA
   <213> Artificial Sequence
<220>
   <221> source
   <222> 1..25
   <223> /mol_type="DNA" /note="BCAN exon 8 reverse primer" /organism="Artificial Sequence"
<400> 36
   ccagagatca tgtgacccag agctt 25
<210> 37
   <211> 25
   <212> DNA
   <213> Artificial Sequence
<220>
   <221> source
   <222> 1..25
   <223> /mol_type="DNA" /note="BCAN exon 9 forward primer" /organism="Artificial Sequence"
<400> 37
   gtgatagctc ccaagacaag gagat 25
<210> 38
   <211> 23
   <212> DNA
   <213> Artificial Sequence
<220>
   <221> source
   <222> 1..23
   <223> /mol_type="DNA" /note="BCAN exon 9 reverse primer" /organism="Artificial Sequence"
<400> 38
   gcagggtcca ggcttcaggt cta 23
<210> 39
   <211> 20
   <212> DNA
   <213> Artificial Sequence
<220>
   <221> source
   <222> 1..20
   <223> /mol_type="DNA" /note="BCAN exon 10 forward primer" /organism="Artificial Sequence"
<400> 39
   ctctgctggc tctctggcat 20
<210> 40
   <211> 21
   <212> DNA
   <213> Artificial Sequence
<220>
   <221> source
   <222> 1..21
   <223> /mol_type="DNA" /note="BCAN exon 10 reverse primer" /organism="Artificial Sequence"
<400> 40
   tgagtgggag agagcaggtg a 21
<210> 41
   <211> 20
   <212> DNA
   <213> Artificial Sequence
<220>
   <221> source
   <222> 1..20
   <223> /mol_type="DNA" /note="BCAN exon 11 forward primer" /organism="Artificial Sequence"
<400> 41
   acggacaggg aagcagggaa 20
<210> 42
   <211> 20
   <212> DNA
   <213> Artificial Sequence
<220>
   <221> source
   <222> 1..20
   <223> /mol_type="DNA" /note="BCAN exon 11 reverse primer" /organism="Artificial Sequence"
<400> 42
   gcggaggcag aagtgcttgg 20
<210> 43
   <211> 20
   <212> DNA
   <213> Artificial Sequence
<220>
   <221> source
   <222> 1..20
   <223> /mol_type="DNA" /note="BCAN exon 12 forward primer" /organism="Artificial Sequence"
<400> 43
   tgggcctcca ctcctcatcc 20
<210> 44
   <211> 22
   <212> DNA
   <213> Artificial Sequence
<220>
   <221> source
   <222> 1..22
   <223> /mol_type="DNA" /note="BCAN exon 12 reverse primer" /organism="Artificial Sequence"
<400> 44
   ttacacacat gcggcttggg tc 22
<210> 45
   <211> 20
   <212> DNA
   <213> Artificial Sequence
<220>
   <221> source
   <222> 1..20
   <223> /mol_type="DNA" /note="BCAN exon 13 forward primer" /organism="Artificial Sequence"
<400> 45
   atcccgggtc tccaggatca 20
<210> 46
   <211> 23
   <212> DNA
   <213> Artificial Sequence
<220>
   <221> source
   <222> 1..23
   <223> /mol_type="DNA" /note="BCAN exon 13 reverse primer" /organism="Artificial Sequence"
<400> 46
   gagcccaggg ctactgttgg ata 23
<210> 47
   <211> 24
   <212> DNA
   <213> Artificial Sequence
<220>
   <221> source
   <222> 1..24
   <223> /mol_type="DNA" /note="BCAN exon 14 forward primer" /organism="Artificial Sequence"
<400> 47
   ccagggctag tgtttggatg agat 24
<210> 48
   <211> 23
   <212> DNA
   <213> Artificial Sequence
<220>
   <221> source
   <222> 1..23
   <223> /mol_type="DNA" /note="BCAN exon 14 reverse primer" /organism="Artificial Sequence"
<400> 48
   ccctcacgtg gtcacttcct atg 23
<210> 49
   <211> 22
   <212> DNA
   <213> Artificial Sequence
<220>
   <221> source
   <222> 1..22
   <223> /mol_type="DNA" /note="HAPLN2 exon 1-2 forward primer" /organism="Artificial Sequence"
<400> 49
   gacattcccc acacacacca ag 22
<210> 50
   <211> 25
   <212> DNA
   <213> Artificial Sequence
<220>
   <221> source
   <222> 1..25
   <223> /mol_type="DNA" /note="HAPLN2 exon 1-2 reverse primer" /organism="Artificial Sequence"
<400> 50
   cgcctgtttc gcttcatagt aattg 25
<210> 51
   <211> 20
   <212> DNA
   <213> Artificial Sequence
<220>
   <221> source
   <222> 1..20
   <223> /mol_type="DNA" /note="HAPLN2 exon 3-5 forward primer" /organism="Artificial Sequence"
<400> 51
   tgtctgccgg ctctccctaa 20
<210> 52
   <211> 25
   <212> DNA
   <213> Artificial Sequence
<220>
   <221> source
   <222> 1..25
   <223> /mol_type="DNA" /note="HAPLN2 exon 3-5 reverse primer" /organism="Artificial Sequence"
<400> 52
   aggatacaga cccatcctga agcac 25
<210> 53
   <211> 46
   <212> DNA
   <213> Artificial Sequence
<220>
   <221> source
   <222> 1..46
   <223> /mol_type="DNA" /note="MLPA CFTRa probe 1" /organism="Artificial Sequence"
<400> 53
   gggttcccta agggttggag ctccattgca atctacctag ccattg 46
<210> 54
   <211> 54
   <212> DNA
   <213> Artificial Sequence
<220>
   <221> source
   <222> 1..54
   <223> /mol_type="DNA" /note="MLPA CFTRa probe 2; 5' phosphate group" /organism="Artificial Sequence"
<400> 54
   gcttatgcct tctctttatc atgaggccgc ttctagattg gatcttgctg gcac 54
<210> 55
   <211> 39
   <212> DNA
   <213> Artificial Sequence
<220>
   <221> source
   <222> 1..39
   <223> /mol_type="DNA" /note="MLPA BCAN PR Probe 1" /organism="Artificial Sequence"
<400> 55
   gggttcccta agggttggag gattgggtgc cctgtttgg 39
<210> 56
   <211> 65
   <212> DNA
   <213> Artificial Sequence
<220>
   <221> source
   <222> 1..65
   <223> /mol_type="DNA" /note="MLPA BCAN PR Probe 2; 5' phosphate group" /organism="Artificial Sequence"
<400> 56
   ctcgccacag ggagccttct gcgcatttcc aagtggtcac tgtctagatt ggatcttgct 60
   ggcac 65
<210> 57
   <211> 49
   <212> DNA
   <213> Artificial Sequence
<220>
   <221> source
   <222> 1..49
   <223> /mol_type="DNA" /note="MLPA BCAN exon 1 probe 1" /organism="Artificial Sequence"
<400> 57
   gggttcccta agggttggac tggtctagcc tctaggaacc gacgcagag 49
<210> 58
   <211> 72
   <212> DNA
   <213> Artificial Sequence
<220>
   <221> source
   <222> 1..72
   <223> /mol_type="DNA" /note="MLPA exon 1 probe 2; 5' phosphate group" /organism="Artificial Sequence"
<400> 58
   gaggcagcgg tagcgtgaca ggctggggaa gagcaaaaat cacggagtct ctagattgga 60
   tcttgctggc ac 72
<210> 59
   <211> 51
   <212> DNA
   <213> Artificial Sequence
<220>
   <221> source
   <222> 1..51
   <223> /mol_type="DNA" /note="MLPA BCAN exon 2 probe 1" /organism="Artificial Sequence"
<400> 59
   gggttcccta agggttggac tatgcaaggt gtggccttag ctgatgccct g 51
<210> 60
   <211> 64
   <212> DNA
   <213> Artificial Sequence
<220>
   <221> source
   <222> 1..64
   <223> /mol_type="DNA" /note="MLPA BCAN exon 2 probe 2; 5' phosphate group" /organism="Artificial Sequence"
<400> 60
   gaaggggaca gctcaggtaa gcaggagccc gaggggtgtc ctctagattg gatcttgctg 60
   gcac 64
<210> 61
   <211> 47
   <212> DNA
   <213> Artificial Sequence
<220>
   <221> source
   <222> 1..47
   <223> /mol_type="DNA" /note="MLPA BCAN exon 3 probe 1" /organism="Artificial Sequence"
<400> 61
   gggttcccta agggttggag ccatctaccg ctgcgaggtc cagcacg 47
<210> 62
   <211> 49
   <212> DNA
   <213> Artificial Sequence
<220>
   <221> source
   <222> 1..49
   <223> /mol_type="DNA" /note="MLPA BCAN exon 3 probe 2; 5' phosphate group" /organism="Artificial Sequence"
<400> 62
   gcatagatga cagcagcgat gccgtatcta gattggatct tgctggcac 49
<210> 63
   <211> 43
   <212> DNA
   <213> Artificial Sequence
<220>
   <221> source
   <222> 1..43
   <223> /mol_type="DNA" /note="MLPA BCAN exon 4 probe 1" /organism="Artificial Sequence"
<400> 63
   gggttcccta agggttggag gggctatgaa cagtgtgatg ctg 43
<210> 64
   <211> 45
   <212> DNA
   <213> Artificial Sequence
<220>
   <221> source
   <222> 1..45
   <223> /mol_type="DNA" /note="MLPA BCAN exon 4 probe 2; 5' phosphate group" /organism="Artificial Sequence"
<400> 64
   gctggctatc tgaccagacc gttctagatt ggatcttgct ggcac 45